# FASCICULE DE BREVET EUROPEEN

(11) **EP 0 874 841 B1**
(45) Date de publication et mention de la délivrance du brevet: **04.10.2001**
(21) Numéro de dépôt: 96943182.4
(22) Date de dépôt: 23.12.1996
(51) Int. Cl.: C07D 401/12, A61K 31/47, C07D 401/14

(54) **DERIVES DE 1-BENZENESULFONYLPYRROLIDINE COMME ANTAGONISTES DU RECEPTEUR DE LA BRADYKININE**
1-BENZOLSULFONYLPYRROLIDINDERIVATE ALS BRADYKININ REZEPTOR ANTAGONISTEN
1-BENZENESULFONYL PYRROLIDINE DERIVATIVES AS BRADYKININ RECEPTOR ANTAGONISTS

(30) Priorité: 29.12.1995 FR 9515706
(43) Date de publication de la demande: 04.11.1998
(73) Titulaire: FOURNIER INDUSTRIE ET SANTE, F-75008 Paris (FR)
(72) Inventeur: DODEY, Pierre, F-21121 Fontaine-lès-Dijon (FR); BONDOUX, Michel, F-21121 Fontaine-lès-Dijon (FR); HOUZIAUX, Patrick, F-78580 Bazemont (FR); BARTH, Martine, F-78490 Montfort-l'Amaury (FR); OU, Khan, F-21121 Hauteville-lès-Dijon (FR)
(74) Mandataire: Hubert, Philippe
(86) Numéro de dépôt international: FR9602066
(87) Numéro de publication internationale: WO9724349

(56) Documents cités:
- EP-A- 0 622 361
- US-A- 5 288 725

## Description

### Domaine de l'invention

La présente invention a trait à de nouveaux composés appartenant à la famille des benzènesulfonamides et plus particulièrement à des composés de 1-benzènesulfonylpyrrolidine, leur procédé de préparation et leur utilisation en thérapeutique.

Ces nouveaux composés présentent notamment une action antagoniste du récepteur B₂ de la bradykinine et sont utiles en thérapeutique, particulièrement pour le traitement de la douleur, de l'inflammation, de l'asthme et des rhinites allergiques.

### Art antérieur

On sait que l'une des possibilités de traitement de certaines pathologies à caractère douloureux et/ou inflammatoire (telles que l'asthme, la rhinite, le choc septique, la douleur dentaire, etc...) est d'inhiber l'action de certaines hormones telles que la bradykinine ou la kallidine. En effet ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour créer des composés susceptibles d'être antagonistes de récepteurs de la bradykinine. La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui interagissent avec les récepteurs de la bradykinine : certains comme le Bradycor (CP.0127 de la société Cortech), l'Icatibant (HOE 140 de la société Hoechst) ["Bradycor" et "Icatibant" sont des dénominations communes internationales (DCl)] ou encore le NPC 17761 (de la société Scios-Nova) présentent une action inhibitrice de la fixation de la bradykinine sur le récepteur B₂ de la bradykinine. Plus récemment, des composés non peptidiques ont été proposés comme antagonistes vis-à-vis de la fixation de la bradykinine sur son récepteur B₂, notamment dans les demandes de brevet publiées EP-A-0596406 et EP-A-0622361. On sait en outre que certains composés de structure apparentée à celle des composés visés dans les deux demandes de brevet précitées ont déjà été décrits, notamment dans DE-A-3617183 et dans EP-A-0261539, eu égard à leurs éventuelles propriétés antithrombotiques.

### But de l'invention

Il existe un besoin d'atténuer ou de supprimer chez le mammifère et surtout chez l'homme les douleurs et les inflammations.

Pour satisfaire ce besoin, on a recherché une nouvelle solution technique qui soit efficace dans le traitement des algies quelle que soit leur origine, notamment celles liées à des phénomènes inflammatoires, d'une part, et dans le traitement des inflammations, d'autre part.

Selon l'invention, on se propose de fournir une nouvelle solution technique, qui met en oeuvre une fixation compétitive au niveau du récepteur B₂ de la bradykinine entre (i) la bradykinine et les hormones apparentées ou analogues telles que la kallidine, et (ii) une substance antagoniste, et qui fait appel à des composés de benzènesulfonamide structurellement différents des produits connus précités et qui limitent ou inhibent substantiellement la fixation de la bradykinine et desdites hormones analogues sur ledit récepteur B₂ de la bradykinine.

Conformément à cette nouvelle solution technique on se propose de fournir, selon un premier aspect de l'invention, des composés de benzènesulfonamide en tant que produits industriels nouveaux ; selon un second aspect de l'invention, un procédé de préparation de ces composés; et selon un troisième aspect de l'invention, l'utilisation de ces composés notamment en thérapeutique en tant qu'ingrédients actifs antalgiques et/ou anti-inflammatoires.

### Objet de l'invention

Selon la nouvelle solution technique de l'invention, on préconise en tant que produit industriel nouveau, un composé de benzènesulfonylpyrrolidine qui est caractérisé en ce qu'il est choisi parmi l'ensemble constitué par :
(i) les composés de formule : 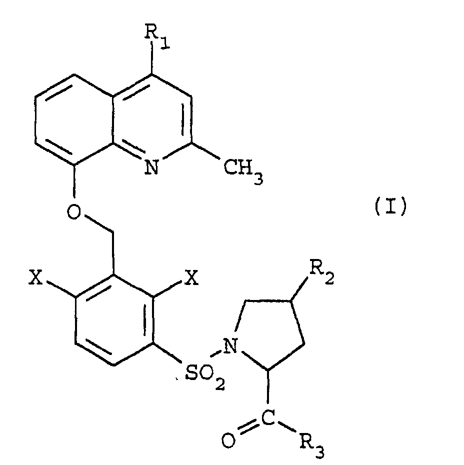 dans laquelle :
   X représente un atome d'halogène,
   R₁ représente un atome d'hydrogène ou un groupe alkyle en C₁-C₃ linéaire ou ramifié,
   R₂ représente un atome d'hydrogène ou un groupe OH,
   R₃ représente un groupe : 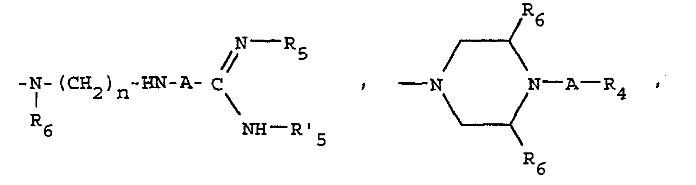 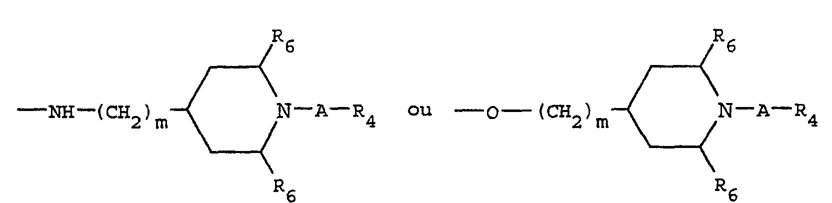
   A représente une liaison simple ou un groupe -CO-(CH₂)ₚ-NH-,
   R₄ représente un atome d'hydrogène ou un groupe 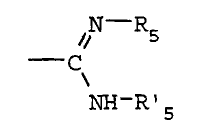
   R₅ et R'₅, identiques ou différents, représentent chacun un atome d'hydrogène ou un groupe alkyle en C₁-C₆ linéaire, ramifié ou cyclisé,
   R₆ représente un atome d'hydrogène ou un groupe alkyle en C₁-C₃ linéaire ou ramifié,
   m est un nombre ayant pour valeur 0, 1 ou 2,
   n est un nombre ayant pour valeur 2, 3 ou 4,
   p est un nombre ayant pour valeur 1, 2 ou 3 ; et,
(ii) leurs sels d'addition.

Selon l'invention, on préconise aussi un procédé de préparation des composés de formule I et de leurs sels d'addition.

On préconise également l'utilisation d'une substance choisie parmi les composés de formule I et leurs sels d'addition non-toxiques pour l'obtention d'un médicament destiné à une utilisation en thérapeutique vis-à-vis des pathologies impliquant la bradykinine ou ses analogues, en particulier vis-à-vis des algies, et notamment dans le traitement ou la prévention de pathologies liées à des états inflammatoires ou douloureux.

### Description détaillée de l'invention

Dans la formule générale I des composés de l'invention, on entend par atome d'halogène un atome de fluor, de chlore, de brome ou d'iode, l'halogène préféré étant l'atome de chlore.

Par groupe alkyle en C₁-C₃ à chaîne hydrocarbonée linéaire ou ramifiée, on entend ici les groupes méthyle, éthyle, propyle et 1-méthyléthyle. Par groupes alkyle en C₁-C₆ linéaire, ramifié ou cyclisé, on entend notamment les groupes méthyle, éthyle, propyle, butyle, pentyle, hexyle, 1-méthyléthyle, 1-méthylpropyle, cyclopentyle et cyclohexyle.

Dans le composé de formule I, l'hétérocycle azoté de type pyrrolidine porteur des groupes COR₃ et R₂ peut comprendre 1 ou 2 atomes de carbone asymétriques. Selon l'invention, ces atomes de carbone peuvent être de configuration (R,S), R ou S ; de préférence, le carbone porteur du groupe COR₃ sera de configuration S.

Par "sels d'addition", on entend ici les sels d'addition d'acide obtenus par réaction d'un composé de formule I avec un acide minéral ou organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, maléique, fumarique, oxalique, citrique et trifluoroacétique.

Par température ambiante (RT) on entend ici une température comprise entre 15 et 25°C, et par température proche de RT une température comprise entre 5 et 35°C.

Le procédé de préparation des composés de formule I et de leurs sels d'addition, que l'on préconise selon l'invention, comprend les étapes consistant à :
1) faire réagir un composé de formule : 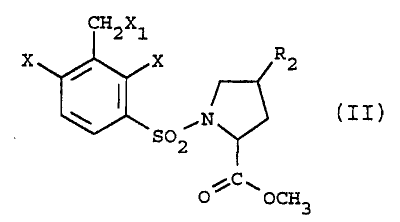
   dans laquelle X et X₁ représentent chacun un halogène,
   R₂ représente un atome d'hydrogène ou un groupe OH, et, le carbone porteur du groupe COOCH₃ et le carbone porteur du groupe
   R₂ lorsque ce dernier n'est pas l'atome d'hydrogène, sont indépendamment l'un de l'autre de configuration (R,S), (R) ou (S), avec un composé de formule : 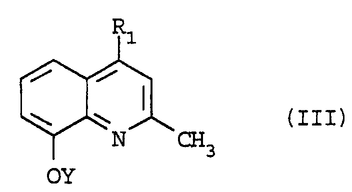 dans laquelle :
      R₁ représente un atome d'hydrogène ou un groupe alkyle en C₁-C₃, et
      Y représente un métal alcalin tel que le sodium ou le potassium,
   dans un solvant anhydre, comme par exemple le diméthylformamide, à température comprise entre 0 et 50° C, pendant 0,5 à 5 heures, pour obtenir un composé de formule : 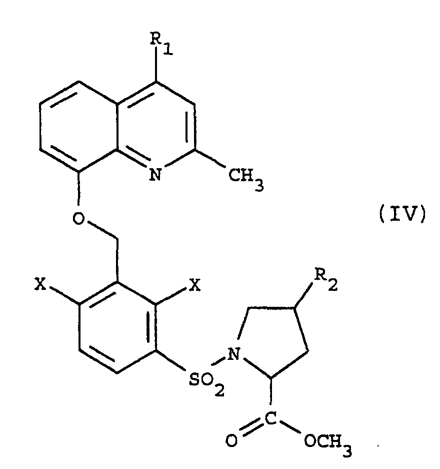 dans laquelle X, R₁ et R₂ conservent la même signification que dans les composés de départ, et les atomes de carbone porteurs de substituants COOCH₃ et R₂ conservent la même configuration que dans le composé II ci-dessus ;
2) soumettre le composé IV, ainsi obtenu, à une hydrolyse alcaline de la liaison ester, par action d'une solution aqueuse d'hydroxyde métallique (notamment NaOH) dans un solvant inerte, notamment le diméthoxyéthane, à une température comprise entre 10 et 50° C, pendant 1 à 30 heures pour obtenir, après acidification, le composé acide de formule : 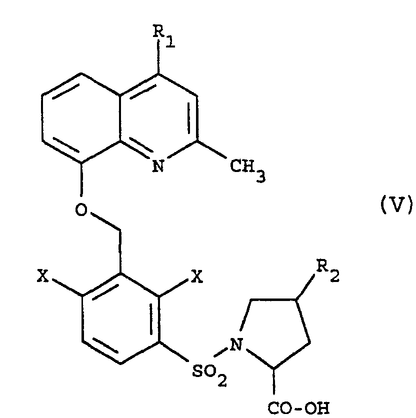 dans laquelle X, R₁ et R₂ conservent la même signification que dans le composé IV ci-dessus ;
3) faire réagir l'acide V, ainsi obtenu, avec un alcool ou une amine de formule: 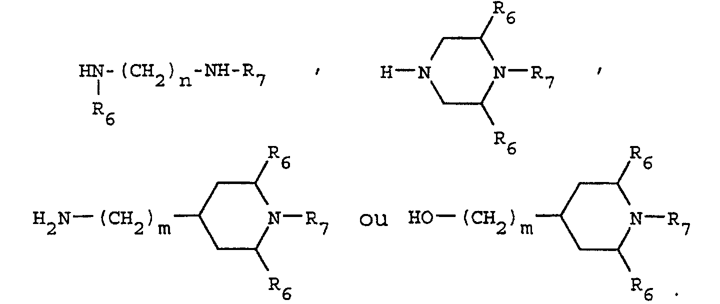 dans lesquelles :
   m représente 0, 1 ou 2,
   n représente 2, 3 ou 4,
   R₆ représente un atome d'hydrogène ou un groupe alkyle en C₁-C₃,
   R₇ représente un groupe amino-protecteur comme par exemple un groupe Boc [(1,1-diméthyléthoxy)carbonyle], ou, dans certains cas,
   un atome d'hydrogène,
   dans un solvant, comme par exemple le dichlorométhane, en présence d'un ou plusieurs activateurs, comme par exemple le 1-hydroxy-7-aza-benzotriazole (HOAT) et le chlorhydrate de 1-[3-(diméthylaminopropyl)-3-éthyl]carbodiimide (EDCI), à une température proche de la température ambiante, pendant 2 à 50 heures, pour obtenir un composé de formule : 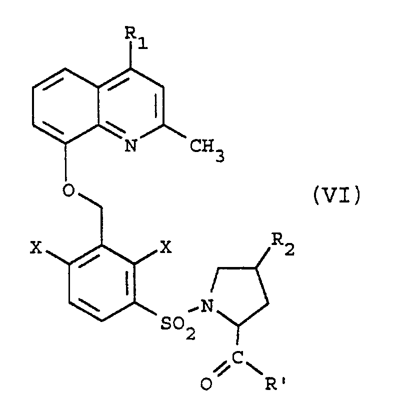 dans laquelle :
   X, R₁ et R₂ conservent la même signification que ci-dessus et R' représente l'un des groupes : 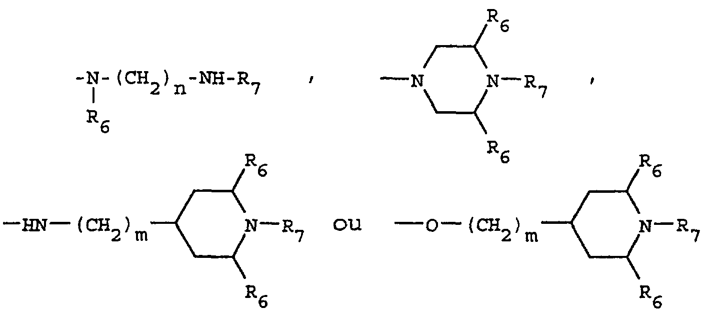 où m, n, R₆ et R₇ conservent la même signification que ci-dessus ;
4) si nécessaire, c'est-à-dire lorsque R₇ représente un groupe amino-protecteur, effectuer la déprotection du composé de formule VI ainsi obtenu à l'étape précédente, par réaction dudit composé de formule VI avec un acide comme par exemple l'acide trifluoroacétique ou l'acide chlorhydrique, éventuellement en présence d'un capteur de radicaux libres comme par exemple l'anisole, et éventuellement dans un solvant comme par exemple l'acétate d'éthyle, à température proche de l'ambiante et pendant 1 à 20 heures, pour obtenir le composé de formule générale VI décrit ci-dessus où R₇ représente un atome d'hydrogène (correspondant au composé de formule I dans laquelle A est une liaison simple et R₄ un atome d'hydrogène) ;
5) si nécessaire, faire réagir le composé de formule VI, ainsi obtenu, où R₇ est un atome d'hydrogène, avec un composé de formule :

   R₈-NH-(CH₂)ₚ-COOH

   dans laquelle p représente un nombre égal à 1, 2 ou 3, et
   R₈ représente un groupe aminoprotecteur du type oxycarbonyle, notamment un groupe alkyloxycarbonyle tel que Boc [(1,1-diméthyléthoxy)carbonyle], dans des conditions de réaction analogues à celles décrites à l'étape 3 ci-dessus, pour obtenir un composé de formule : 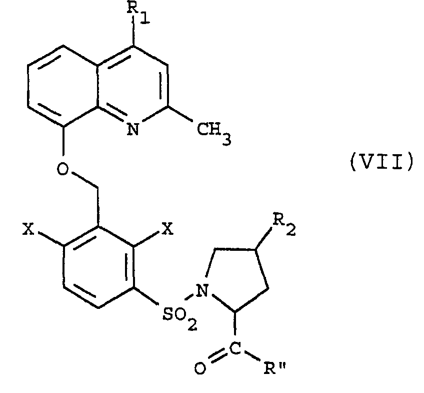 dans laquelle X, R₁ et R₂ conservent la même signification que ci-dessus et R" représente un groupe 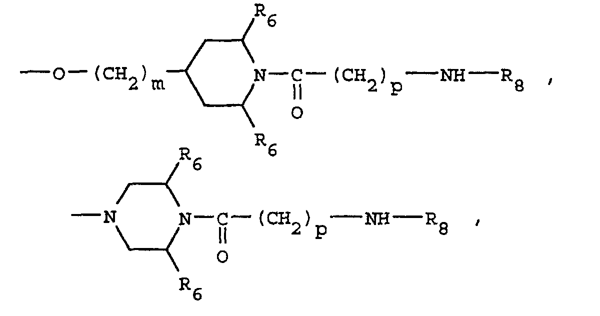 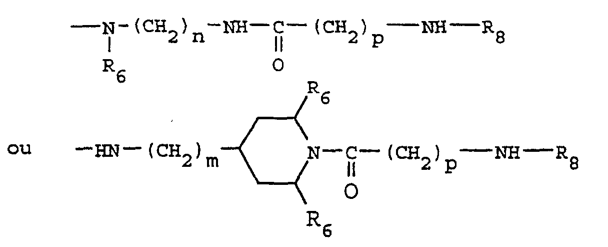 où m, n, p, R₆ et R₈ conservent la même signification que ci-dessus,
6) faire réagir le composé de formule VII, obtenu ci-dessus selon l'étape 5, dans des conditions analogues à celles décrites à l'étape 4 ci-dessus, de façon à remplacer le groupe aminoprotecteur R₈ par un atome d'hydrogène et obtenir un composé de formule : 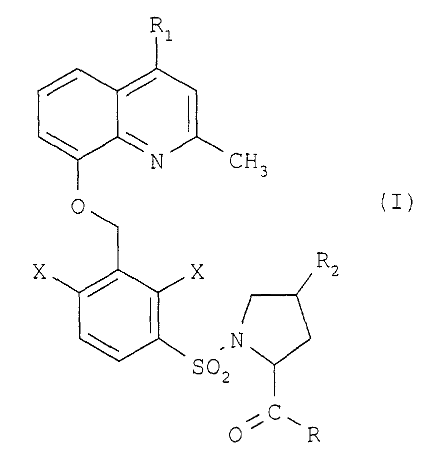 dans laquelle X, R₁ et R₂ conservent la même signification que ci-dessus et R représente un groupe 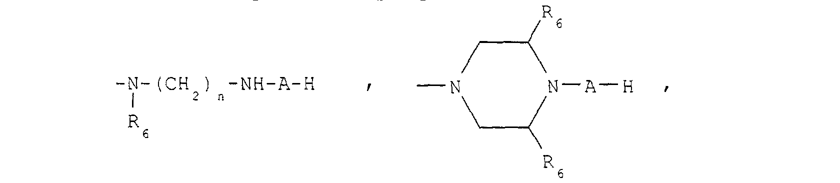 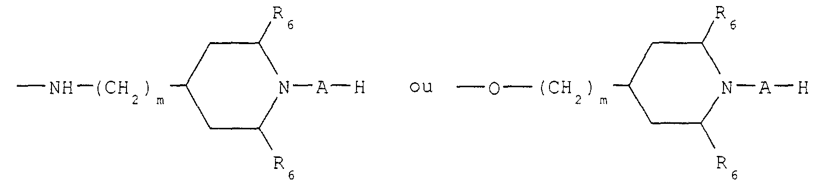 dans lesquels m, n et R₆ conservent la même signification que ci-dessus et A représente le groupe -CO- (CH₂)ₚ-NH-, où p est un nombre égal 1, 2 ou 3,
7) si nécessaire, faire réagir un composé de formule I obtenu à l'une des étapes 4 ou 6 ci-dessus, où A représente une liaison simple ou un groupe 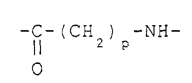 avec un composé de formule 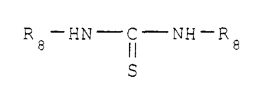 dans laquelle R₈ représente un groupe amino-protecteur de type oxycarbonyle, notamment un groupe alkyloxycarbonyle tel que Boc [(1,1-diméthyléthoxy)-carbonyle], dans un solvant comme par exemple la diméthylformamide, en présence d'une base comme par exemple la triéthylamine et en présence de chlorure mercurique, à une température comprise entre 0 et 30 °C, pendant 1 à 6 heures, pour obtenir le composé de formule : 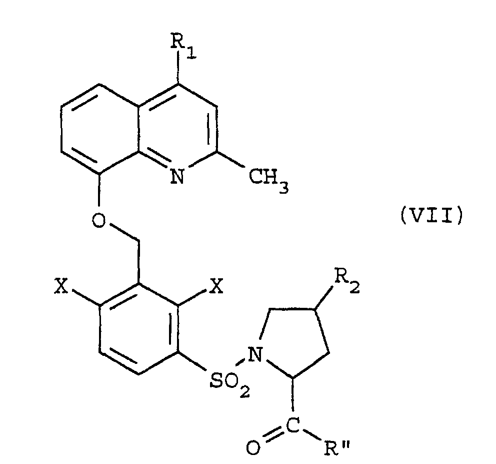 dans laquelle R₁, R₂ et X conservent la même signification que ci-dessus et R" représente un groupe 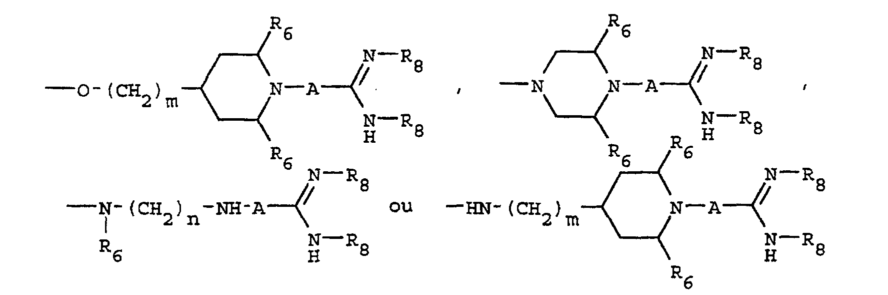 dans lesquels
   A représente une liaison simple ou le groupe -CO- (CH₂)ₚ-NH-,
   et n, m, p, R₆ et R₈ conservent la même signification que ci-dessus ; et,
8) déprotéger le composé de formule VII, ainsi obtenu selon l'étape 7, suivant une réaction analogue à celle de l'étape 6 ci-dessus, pour remplacer le groupe amino-protecteur R₈ par un atome d'hydrogène et obtenir ainsi un composé de formule I selon l'invention où R₄ représente un groupe -C(=NR₅)NHR'₅ et R₅ et R'₅ représentent chacun un atome d'hydrogène.

En première variante, l'étape 7 peut être réalisée comme indiqué ci-après, sans que la déprotection de l'étape 8 soit effectuée :
7a) faire réagir le chlorhydrate d'un composé de formule I obtenu à l'une des étapes 4 ou 6 ci-dessus, avec un composé de formule :

R₅-N=C=N-R'₅

dans laquelle R₅ et R'₅ représentent chacun un groupe alkyle en C₁-C₆ linéaire, ramifié ou cyclisé,
dans un solvant inerte comme par exemple l'acétonitrile, à une température proche de la température ambiante, pendant 4 à 48 heures, pour obtenir un composé de formule I, selon l'invention, où R₅ et R'₅ représentent chacun un groupe alkyle en C₁-C₆ linéaire, ramifié ou cyclisé, et A est une liaison simple ou le groupe -CO- (CH₂)ₚ-NH-.

Selon une seconde variante de l'étape 7, on fait réagir le chlorhydrate d'un composé de formule VI obtenu selon l'une des étapes 4 ou 6 ci-dessus avec un composé de formule 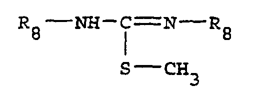 dans laquelle R₈ représente un groupe amino-protecteur du type Boc,
en présence d'une base organique aprotique telle que la triéthylamine et d'oxyde mercurique, dans un solvant comme par exemple l'éthanol, à température ambiante pendant 5 à 50 heures, pour obtenir le composé de formule VII, de structure analogue à celle obtenue à l'étape 7 ci-dessus. Ce composé est ensuite traité suivant le procédé décrit à l'étape 8 ci-dessus pour conduire au composé de formule I selon l'invention, dans lequel
A représente une liaison simple ou un groupe -CO- (CH₂)ₚ-NH-, et R₄ représente le groupe -C(=NH)NH₂.

Certains composés de formule III et IV, en particulier les composés visés dans la revendication 3 ci-après, sont des produits nouveaux utiles en tant qu'intermédiaires de synthèse et constituent l'un des objets de l'invention.

L'invention sera mieux comprise à la lecture des exemples de préparation qui suivent et des résultats d'essais pharmacologiques obtenus avec certains composés selon l'invention. Dans le cas de composés présentant dans leur structure un carbone asymétrique, l'absence d'indication particulière ou la mention (R,S) [ou (D,L) dans le cas d'acides aminés] signifie qu'il s'agit de composés racémiques ; dans le cas de composés présentant une chiralité, celle-ci est indiquée à la suite immédiate de l'indexation du substituant porté par ledit carbone asymétrique; on utilise alors les signes (R) ou (S), conformément aux règles de Cahn, Ingold et Prelog, ou, dans le cas d'acides aminés les mentions (D) ou (L). La nomenclature utilisée dans les exemples est celle préconisée par les Chemical Abstracts : ainsi certains dérivés de la L-proline peuvent devenir, après réaction de la fonction acide avec une amine, des dérivés de 2-(S)-pyrrolidinecarboxamide.

Dans la partie expérimentale, les "préparations" sont relatives aux composés intermédiaires et les "exemples" sont relatifs aux produits selon l'invention.

Les points de fusion (F) indiqués ci-après sont en général mesurés à l'aide d'un banc Koffler et ne sont pas corrigés, il s'agit alors de points de fusion instantanée.

### PREPARATION I

### Chlorure de 3-bromométhyl-2,4-dichlorobenzènesulfonyle

A une solution à température ambiante de 41,52 g (0,16 mole) de chlorure de 2,4-dichloro-3-méthylbenzènesulfonyle dans 150 ml de 1,1,2,2-tétrachloroéthane on ajoute 85,44 g (0,48 mole) de N-bromosuccinimide puis 200 mg de peroxyde de benzoyle. Le mélange réactionnel est porté à 120°C pendant 2 heures. On refroidit, filtre et lave le filtrat successivement avec de l'eau, une solution saturée de bicarbonate de sodium et enfin de l'eau jusqu'à neutralité. Ensuite, la phase organique que l'on recueille du filtrat est séchée sur sulfate de magnésium et concentrée. Par recristallisation dans l'hexane, on obtient 25,53 g du produit attendu, sous forme de cristaux blancs (Rendement = 47 %).
**F = 90-92°C**

### PREPARATION II

### Chlorure de 3-chlorométhyl-2,4-dichlorobenzènesulfonyle

A une solution de 6,5 g (0,025 mole) de chlorure de 2,4-dichloro-3-méthylbenzènesulfonyle dans 30 ml de 1,1,2,2-tétrachloroéthane, à température ambiante et sous atmosphère d'azote, on ajoute 10 g (0,075 mole) de N-chlorosuccinimide et 30 mg de peroxyde de benzoyle. Le mélange réactionnel est porté à 120°C pendant 3 heures, refroidi à température ambiante, versé sur de l'eau, puis extrait avec du dichlorométhane. La phase organique est lavée avec de l'eau, une solution saturée de bicarbonate de sodium et enfin de l'eau jusqu'à neutralité. Elle est ensuite séchée sur sulfate de magnésium et concentrée sous pression réduite. Par recristallisation dans l'hexane, on obtient 0,85 g du produit attendu, sous forme de cristaux blancs (Rendement = 11,5 %).
**F = 68°C**

### PRÉPARATION III

### N-[(3-bromométhyl-2,4-dichlorophényl)sulfonyl]-(D,L)-proline, méthyl ester

A une solution de 13,24 g (0,08 mole) de chlorhydrate de (D,L)-proline, méthyl ester dans 60 ml de dichlorométhane, on ajoute 27,08 g (0,08 mole) de composé obtenu selon la préparation I. On refroidit à 0°C et additionne goutte à goutte une solution de 23,3 ml (0,16 mole) de triéthylamine dans 20 ml de dichlorométhane. On laisse agiter ensuite à température ambiante pendant 30 minutes. Le mélange réactionnel est versé sur de l'eau et extrait au dichlorométhane. La phase organique est lavée par une solution d'acide chlorhydrique 1N puis à l'eau jusqu'à pH neutre. Elle est enfin séchée et concentrée sous pression réduite. On obtient 34,48 g d'huile utilisée directement dans les étapes ultérieures. Le produit obtenu contient également le composé N-[(3-chlorométhyl-2,4-dichlorophényl)sulfonyl]-(D,L)-proline, méthyl ester provenant d'une réaction secondaire. Ce sous-produit possédant sensiblement la même réactivité que le dérivé bromé, on utilise directement le mélange, sans purification complémentaire.

### PREPARATION IV

### N-[(3-bromométhyl-2,4-dichlorophényl)sulfonyl]-L-proline, méthyl ester

En opérant de façon analogue à la préparation III, au départ du chlorhydrate de L-proline, méthyl ester, on obtient le produit attendu sous forme d'une huile. (Le produit obtenu contient l'analogue chloré issu d'une réaction secondaire d'échange entre halogène).

### PREPARATION V

### N-[(3-bromométhyl-2,4-dichlorophényl)sulfonyl]-4-(trans)-hydroxy-L-proline, méthyl ester

En opérant de façon analogue à la préparation III, au départ du chlorhydrate de 4-(trans)-hydroxy-L-proline, méthyl ester, on obtient le produit attendu sous forme d'une huile. (Le produit obtenu contient l'analogue chloré issu d'une réaction secondaire d'échange entre halogène).

### PREPARATION VI

### N-[[3-[(2-méthylquinolin-8-yl)oxyméthyl]-2,4-dichlorophényl]sulfonyl]-D,L-proline, méthyl ester

A une solution de 0,270 g (1,7.10⁻³ mole) de 8-hydroxy-2-méthylquinoléine dans 5 ml de N,N-diméthylformamide (DMF), on ajoute 0,051 g (1,7.10⁻³ mole) d'hydrure de sodium à 80 % en suspension dans de l'huile. Après agitation pendant dix minutes à température ambiante on ajoute 0,8 g du composé obtenu selon la préparation III en solution dans 2 ml de DMF. L'agitation est maintenue 2 heures à température ambiante. Le mélange réactionnel est étendu d'eau et extrait à l'acétate d'éthyle. Les phases organiques sont lavées à l'eau, séchées sur sulfate de sodium, filtrées et concentrées sous pression réduite. Le résidu obtenu est purifié par chromatographie sur gel de silice en éluant à l'aide d'un mélange toluène/acétate d'éthyle (80/20 ; v/v). Le solide récupéré est recristallisé dans 15 ml d'isopropanol pour donner 0,7 g du produit attendu, sous forme d'une poudre blanche (rendement = 81 %).
**F = 142°C**

### PREPARATION VII

### N-[[3-[(2-méthylquinolin-8-yl)oxyméthyl]-2,4-dichlorophényl]sulfonyl]-L-proline, méthyl ester

En opérant de façon analogue à la préparation VI, au départ du composé obtenu selon la préparation IV, on obtient le produit attendu avec un rendement de 83 % après recristallisation dans l'isopropanol.
**F = 136°C**
**[α]**_{**D**}^{**23**} **= +29,4° (c = 1,01 ; CHCl**_{**3**}**)**

### PREPARATION VIII

### N-[[3-[(2,4-diméthylquinolin-8-yl)oxyméthyl]-2,4-dichlorophényl]sulfonyl]-L-proline, méthyl ester

En opérant de façon analogue à la préparation VI, au départ du composé obtenu selon la préparation IV et de 2,4-diméthyl-8-hydroxyquinoléine, on obtient le produit attendu avec un rendement de 72 % après recristallisation dans l'isopropanol.
**F = 138°C**
**[α]**_{**D**}^{**22**} **= -28° (c = 0,5 ; CHCl**_{**3**}**)**

### PREPARATION IX

### N-[[3-[(2,4-diméthylquinolin-8-yl)oxyméthyl]-2,4-dichlorophényl]sulfonyl]-4-(trans)-hydroxy-L-proline, méthyl ester

En opérant de façon analogue à la préparation VI, au départ du composé obtenu selon la préparation V et de 2,4-diméthyl-8-hydroxyquinoléine, et après purification par chromatographie sur gel de silice en éluant avec un mélange dichlorométhane/acétate d'éthyle (7/3 ; v/v), puis cristallisation dans l'éther isopropylique, on obtient le produit attendu avec un rendement de 44 %, sous forme d'un solide blanc.
**F = 100°C**
**[α]**_{**D**}^{**21**} **= -30° (c = 0,31 ; CHCl**_{**3**}**)**

### PREPARATION X

### N-[[3-[(2-méthylquinolin-8-yl)oxyméthyl]-2,4-dichlorophényl]sulfonyl]-D,L-proline

A une solution de 5,14 g (10,2.10⁻³ mole) du composé obtenu selon la préparation VI, dans 100 ml de 1,2-diméthoxyéthane, on ajoute 20 ml (20.10⁻³ mole) d'une solution aqueuse d'hydroxyde de sodium 1N. Le mélange réactionnel est agité à 40°C pendant 1,5 heure, puis à température ambiante pendant 20 heures. Le mélange est ensuite concentré sous pression réduite et le résidu est repris à l'eau et acidifié jusqu'à pH5 à l'aide d'acide chlorhydrique 1N. Après extraction à l'aide de dichlorométhane, la phase organique est lavée à l'eau, séchée et concentrée sous pression réduite. Le solide ainsi obtenu est recristallisé dans 30 ml d'isopropanol pour donner 4,4 g du produit attendu, sous forme de cristaux blancs (rendement = 87 %).
**F = 120°C**

### PREPARATION XI

### N-[[3-[(2-méthylquinolin-8-yl)oxyméthyl]-2,4-dichlorophényl]sulfonyl]-L-proline

En opérant de façon analogue à la préparation X, au départ du composé obtenu selon la préparation VII, on obtient le produit attendu avec un rendement de 93 %.
**F = 135°C**
**[α]**_{**D**}^{**23**} **= -99,3° (c = 0,31 ; CHCl**_{**3**}**)**

### PREPARATION XII

### N-[[3-[(2,4-diméthylquinolin-8-yl)oxyméthyl]-2,4-dichlorophényl]sulfonyl]-L-proline

En opérant de façon analogue à la préparation X, au départ du composé obtenu selon la préparation VIII, on obtient le produit attendu avec un rendement de 95 %.
**F = 219-220°C**
**[α]**_{**D**}^{**22**} **= -79,2° (c = 0,31 ; CHCl**_{**3**}**)**

### PREPARATION XIII

### N-[[3-[(2,4-diméthylquinolin-8-yl)oxyméthyl]-2,4-dichlorophényl]sulfonyl]-4-(trans)-hydroxy-L-proline

On prépare une suspension de 0,53g (0,983.10⁻³ mole) du composé obtenu selon la préparation IX dans 35 ml de méthanol, on ajoute 5 ml d'une solution de soude 0,3 N et on agite à reflux pendant 18 heures. On concentre ensuite le mélange réactionnel sous pression réduite puis on reprend le résidu avec de l'eau et on acidifie jusqu'à pH5 à l'aide d'acide chlorhydrique 1N. Après extraction avec du dichlorométhane, la phase organique est lavée à l'eau, séchée sur sulfate de magnésium puis concentrée sous pression réduite. On obtient ainsi 490 mg du produit attendu, sous forme d'un solide blanc.
**F > 260°C**
**[α]**_{**D**}^{**21**} **= -55° (c = 0,36 ; CHCl**_{**3**}**)**

### PREPARATION XIV

### 1-[[3-[(2-méthylquinolin-8-yl)oxyméthyl]-2,4-dichloro-phényl]sulfonyl]-N-[2-(1,1-diméthyléthoxycarbonylamino)éthyl]-N-méthyl-2-(S)-pyrrolidinecarboxamide

On prépare une solution de 0,742 g (1,5.10⁻³ mole) de l'acide obtenu selon la préparation XI, dans 10 ml de dichlorométhane et on ajoute 0,21 g (1,1.10⁻³ mole) de chlorhydrate de 1-(3-diméthylaminopropyl)-3-éthyl-carbodiimide (EDCI), 0,15 g (1,1.10⁻³ mole) de 1-hydroxy-7-azabenzotriazole (HOAT), puis 0,261g (1,5.10⁻³ mole) de N-méthyl-N'-(1,1-diméthyléthoxycarbonyl)-1,2-éthanediamine. Le mélange réactionnel est agité à température ambiante pendant 20 heures puis concentré sous pression réduite. Le résidu obtenu est purifié par chromatographie sur gel de silice en éluant avec un mélange toluène/isopropanol (95/5 ; v/v). On obtient 0,61g du produit attendu, sous forme d'un solide blanc avec un rendement de 61 %.
**F = 88°C**
**[α]**_{**D**}^{**22**} **= -4,5° (c=0,2 ; CH**_{**3**}**OH)**
**[α]**_{**D**}^{**22**} **= -4,5° (c=0,2 ; CH**_{**3**}**OH)**

### PREPARATION XV

### 1-[[3-[(2-méthylquinolin-8-yl)oxyméthyl]-2,4-dichlorophényl]sulfonyl]-N-[3-(1,1-diméthyléthoxycarbonylamino)propyl]-2-(S)-pyrrolidinecarboxamide

En opérant de façon analogue à la préparation XIV, au départ de N-(1,1-diméthyléthoxycarbonyl)-1,3-propanediamine, on obtient le produit attendu avec un rendement de 57 %.
**F = 78-80°C**
**[α]**_{**D**}^{**22**} **= -38°(c = 0,3 ; CH**_{**3**}**OH)**

### PREPARATION XVI

### 1-[[3-[(2-méthylquinolin-8-yl)oxyméthyl]-2,4-dichlorophényl]sulfonyl]-N-[[1-(1,1-diméthyléthoxycarbonyl)pipéridin-4-yl]méthyl]-2-(S)-pyrrolidinecarboxamide

En opérant de façon analogue à la préparation XIV, au départ de 1-(1,1-diméthyléthoxycarbonyl)-4-(aminométhyl)pipéridine, on obtient le produit attendu avec un rendement de 20%.
**F = 68°C**
**[α]**_{**D**}^{**22**} **= +38° (c = 0,32 ; CH**_{**3**}**OH)**

### PREPARATION XVII

### 1-[[3-[(2-méthylquinolin-8-yl)oxyméthyl]-2,4-dichlorophényl]sulfonyl]-N-[1-(1,1-diméthyléthoxycarbonyl)pipéridin-4-yl]-2-(S)-pyrrolidinecarboxamide

En opérant de façon analogue à la préparation XIV, au départ de 1-(1,1-diméthyléthoxycarbonyl)-4-aminopipéridine, on obtient le produit attendu avec un rendement de 42,3 %.
**F = 70-74°C**
**[α]**_{**D**}^{**22**} **= -27° (c = 0,37 ; CH**_{**3**}**OH)**

### PREPARATION XVIII

### Acide 1-[[3-[(2-méthylquinolin-8-yl)oxyméthyl]-2,4-dichloro-phényl]-sulfonyl]-2-(S)-pyrrolidinecarboxylique, 1-(1,1-diméthyléthoxycarbonyl)pipéridin-4-yl ester

En opérant de façon analogue à la préparation XIV, au départ de 1-(1,1-diméthyléthoxycarbonyl)-4-hydroxypipéridine, on obtient le produit attendu sous forme d'une huile avec un rendement de 23,6%.
**[α]**_{**D**}^{**22**} **= +22° (c = 0,17 ; CH**_{**3**}**OH)**

### PREPARATION XIX

### Acide 1-[[3-[(2-méthylquinolin-8-yl)oxyméthyl]-2,4-dichlorophényl]sulfonyl]-2-(S)-pyrrolidinecarboxylique, [1-(1,1-diméthyléthoxycarbonyl)pipéridine-4-yl-méthyl] ester

En opérant de façon analogue à la préparation XIV, au départ de 1-(1,1-diméthyléthoxycarbonyl)-4-hydroxyméthylpipéridine, on obtient le produit attendu avec un rendement de 37,6 %.
**F = 66-68°C**
**[α]**_{**D**}^{**22**} **= -19° (c = 0,28 ; CH**_{**3**}**OH)**

### PREPARATION XX

### 1-[[3-[(2,4-diméthylquinolin-8-yl)oxyméthyl]-2,4-dichlorophényl]sulfonyl]-N-[3-(1,1-diméthyléthoxycarbonylamino)propyl]-2-(S)-pyrrolinecarboxamide

En opérant de façon analogue à la préparation XIV, au départ du composé de la préparation XII et de N-(1,1-diméthyléthoxycarbonyl)-1,3-propanediamine, on obtient le produit attendu avec un rendement de 74 %.
**F = 80-84°C**
**[α]**_{**D**}^{**22**} **= -38° (c = 0,32 ; CH**_{**3**}**OH)**

### PREPARATION XXI

### 1-[[3-[(2,4-diméthylquinolin-8-yl)oxyméthyl]-2,4-dichloro-phényl]sulfonyl]-2-(S)-[[4-(1,1-diméthyléthoxycarbonyl)pipérazin-1-yl]carbonyl]pyrrolidine

En opérant de façon analogue à la préparation XX, au départ de 1-(1,1-diméthyléthoxycarbonyl)pipérazine, on obtient le produit attendu avec un rendement de 66 %.
**F = 100°C**
**[α]**_{**D**}^{**24**}**= -33,3° (c = 0,30 ; CH**_{**3**}**OH)**

### PREPARATION XXII

### 1-[[3-[(2,4-diméthylquinolin-8-yl)oxyméthyl]-2,4-dichloro-phényl]sulfonyl]-2-(S)-[[4-(1,1-diméthyléthoxycarbonyl)pipérazin-1-yl]carbonyl]-4-(R)-hydroxypyrrolidine

En opérant de façon analogue à la préparation XXI, au départ du composé de la préparation XIII, on obtient le produit attendu avec un rendement de 72 %.
**F = 223°C**
**[α]**_{**D**}^{**21**}**= -33° (c = 0,34 ; CHCl**_{**3**}**)**

### Exemple 1

### 1-[[3-[(2-méthylquinolin-8-yl)oxyméthyl]-2,4-dichlorophényl]sulfonyl]-2-(S)-[(3,5-diméthylpipérazin-1-yl)carbonyl]pyrrolidine

En opérant de façon analogue à la préparation XIV, au départ d'un excès de 2,6-diméthylpipérazine, on obtient le produit attendu avec un rendement de 13 %.
**F = 102-106°C**
**[α]**_{**D**}^{**22**}**= -2° (c = 0,3 ; CHCl**_{**3**}**)**

### Exemple 2

### 1-[[3-[(2-méthylquinolin-8-yl)oxyméthyl]-2,4-dichlorophényl]sulfonyl]-2-(S)-[(pipérazin-1-yl)carbonyl]pyrrolidine

En opérant de façon analogue à la préparation XIV, au départ d'un excès de pipérazine, on obtient le produit attendu avec un rendement de 20 %.
**F = 164-166°C**
**[α]**_{**D**}^{**24**}**= -1° (c = 0,28 ; CHCl**_{**3**}**)**

### Exemple 3 (exemple préparatif)

### 1-[[3-[(2-méthylquinolin-8-yl)oxyméthyl]-2,4-dichlorophényl]sulfonyl]-N-(2-aminoéthyl)-2-(S)-pyrrolidinecarboxamide

En opérant de façon analogue à la préparation XIV, au départ d'un excès de 1,2-éthanediamine, on obtient le produit attendu avec un rendement de 40,9%.
**F = 78-80°C**
**[α]**_{**D**}^{**21**}**= -58,6° (c = 0,35 ; CHCl**_{**3**}**)**

### Exemple 4 (exemple préparatif)

### 1-[[3-[(2,4-diméthylquinolin-8-yl)oxyméthyl]-2,4-dichlorophényl]sulfonyl]-N-(2-aminoéthyl)-2-(S)-pyrrolidinecarboxamide

En opérant de façon analogue à la préparation XX, au départ d'un excès de 1,2-éthanediamine, on obtient le produit attendu avec un rendement de 42,7 %.
**F = 104-106°C**
**[α]**_{**D**}^{**21**}**= -55,2° (c = 0,28 ; CHCl**_{**3**}**)**

### Exemple 5

### 1-[[3-[(2,4-diméthylquinolin-8-yl)oxyméthyl]-2,4-dichlorophényl]sulfonyl]-2-(S)-[(pipérazin-1-yl)carbonyl]pyrrolidine, bistrifluoroacétate

On prépare un mélange de 0,6 g (0,885.10⁻³ mole) du composé obtenu selon la préparation XXI et de 96 mg (0,885.10⁻³ mole) d'anisol, puis on ajoute à 0°C 3ml d'acide trifluoroacétique. La solution est agitée pendant 3 heures à 0°C puis pendant une nuit à température ambiante. Après concentration sous pression réduite, le résidu est précipité dans de l'éther diéthylique anhydre. Le précipité est séparé, rincé à l'éther et séché sous vide. On obtient ainsi 0,64 g du produit attendu sous forme d'un solide blanc. (Rendement = 90 %).
**F = 150°C**
**[α]**_{**D**}^{**24**}**= +12,6° (c = 0,31 ; CH**_{**3**}**OH)**

### Exemple 6 (exemple préparatif)

### 1-[[3-[(2-méthylquinolin-8-yl)oxyméthyl]-2,4-dichlorophényl]sulfonyl]-N-méthyl-N-(2-aminoéthyl)-2-(S)-pyrrolidinecarboxamide, bistrifluoroacétate

En opérant de façon analogue à l'exemple 5 au départ du composé obtenu selon la préparation XIV, on obtient le produit attendu avec un rendement de 95 %.
**F = 130°C**
**[α]**_{**D**}^{**22**}**= +3,2° (c = 0,5 ; CH**_{**3**}**OH)**

### Exemple 7

### 1-[[3-[(2,4-diméthylquinolin-8-yl)oxyméthyl]-2,4-dichlorophényl]sulfonyl]-4-(R)-hydroxy-2-(S)- [(pipérazin-1-yl)carbonyl]pyrrolidine, bistrifluoroacétate

En opérant de façon analogue à l'exemple 5, au départ du composé obtenu selon la préparation XXII, on obtient le produit attendu avec un rendement de 92 %.
**F = 165°C**
**[α]**_{**D**}^{**23**}**= +19° (c = 0,32 ; CH**_{**3**}**OH)**

### Exemple 8

### 1-[[3-[(2-méthylquinolin-8-yl)oxyméthyl]-2,4-dichlorophényl]sulfonyl]-N-(pipéridin-4-yl)-2-(S)-pyrrolidinecarboxamide, dichlorhydrate

On prépare 50 ml d'une solution 1,25N de chlorure d'hydrogène dans l'acétate d'éthyle, dans laquelle on mélange ensuite 8,25g (12,17.10⁻³ mole) du composé obtenu selon la préparation XVII. Le milieu réactionnel est agité pendant 15 heures à température ambiante. Le précipité obtenu est filtré, lavé à l'éther puis séché et repris en solution dans de l'eau distillée. Après lyophilisation de la solution ainsi obtenue, on obtient 7,11 g du produit attendu sous forme d'un solide blanc (Rendement = 95 %).
**F = 177°C**
**[α]**_{**D**}^{**22**}**= -21° (c = 0,33 ; CH**_{**3**}**OH)**

### Exemple 9 (exemple préparatif)

### 1 -[[3-[(2-méthylquinolin-8-yl)oxyméthyl]-2,4-dichlorophényl]sulfonyl]-N-(3-aminopropyl)-2-(S)-pyrrolidinecarboxamide, dichlorhydrate

En opérant de façon analogue à l'exemple 8, au départ du composé obtenu selon la préparation XV, on obtient le produit attendu avec un rendement de 68 %.
**F = 158-162°C**
**[α]**_{**D**}^{**22**} **= -36° (c = 0,29 ; CH**_{**3**}**OH)**

### Exemple 10

### 1-[[3-[(2-méthylquinolin-8-yl)oxyméthyl]-2,4-dichlorophényl]sulfonyl]-N-[(pipéridin-4-yl)méthyl]-2-(S)-pyrrolidinecarboxamide, dichlorhydrate

En opérant de façon analogue à l'exemple 8, au départ du composé obtenu selon la préparation XVI, on obtient le produit attendu avec un rendement de 59 %.
**F = 170°C**
**[α]**_{**D**}^{**22**}**= -27° (c = 0,26 ; CH**_{**3**}**OH)**

### Exemple 11

### Acide 1-[[3-[(2-méthylquinolin-8-yl)oxyméthyl]-2,4-dichlorophényl]sulfonyl]-2-(S)-pyrrolidinecarboxylique, pipéridin-4-yl ester, dichlorhydrate

En opérant de façon analogue à l'exemple 8, au départ du composé obtenu selon la préparation XVIII, on obtient le produit attendu avec un rendement de 92 %.
**F = 150-154°C**
**[α]**_{**D**}^{**22**}**= +11° (c= 0,3 ; CH**_{**3**}**OH)**

### Exemple 12

### Acide 1-[[3-[(2-méthylquinolin-8-yl)oxyméthyl]-2,4-dichlorophényl]sulfonyl]-2-(S)-pyrrolidinecarboxylique, [(pipéridin-4-yl) méthyl] ester, dichlorhydrate

En opérant de façon analogue à la préparation XXX, au départ du composé obtenu selon la préparation XIX, on obtient le produit attendu avec un rendement de 95 %.
**F = 154-158°C**
**[α]**_{**D**}^{**22**}**= +7° (c = 0,31 ; CH**_{**3**}**OH)**

### Exemple 13 (exemple préparatif)

### 1-[[3-[(2,4-diméthylquinolin-8-yl)oxyméthyl]-2,4-dichlorophényl]sulfonyl]-N-(3-aminopropyl)-2-(S)-pyrrolidinecarboxamide, dichlorhydrate

En opérant de façon analogue à l'exemple 8, au départ du composé obtenu selon la préparation XX, on obtient le produit attendu avec un rendement de 68 %.
**F = 158-162°C**
**[α]**_{**D**}^{**22**}**= -36° (c = 0,29 ; CH**_{**3**}**OH)**

### PREPARATION XXIII

### 1-[[3-[(2,4-diméthylquinolin-8-yl)oxyméthyl]-2,4-dichlorophényl]sulfonyl]-2(S)-[[4-[[(1,1-diméthyléthoxycarbonyl)amino]-[(1,1-diméthyléthoxycarbonyl)imino]méthyl]pipérazin-1-yl]carbonyl]pyrrolidine

a) On traite par une solution de soude 1N une solution de 0,475 g du composé obtenu selon l'exemple 5 pour amener le pH à une valeur légèrement basique et on extrait deux fois par de l'acétate d'éthyle. Les phases organiques rassemblées sont séchées sur sulfate de magnésium et concentrées sous pression réduite. On obtient ainsi le composé correspondant à l'exemple 5 sous forme de base.
b) On prépare un mélange de 0,34g (0,589.10⁻³mole) du composé obtenu ci-dessus, de 0,131g (1,3.10⁻³ mole) de triéthylamine et de 0,17g (0,589.10⁻³ mole) de N,N'-bis(1,1-diméthyléthoxycarbonyl)thiourée dans 10 ml de DMF. On refroidit à 0°C, on ajoute 0,18g (0,65.10-3 mole) de chlorure mercurique, puis on laisse sous puis on laisse sous agitation pendant 3 heures à température ambiante. Le mélange réactionnel est ensuite dilué par 60 ml d'acétate d'éthyle et lavé à l'eau. La phase organique est séchée, puis concentrée sous pression réduite. Le résidu est purifié par chromatographie sur gel de silice en éluant avec un mélange dichlorométhane/acétate d'éthyle (6/4 ; v/v). On obtient ainsi 0,38g du produit attendu sous forme d'un solide blanc (Rendement = 79 %).
   **F = 100°C**
   **[α]**_{**D**}^{**24**} **= -25° (c = 0,29 ; CH**_{**3**}**OH)**

### PREPARATION XXIV

### 1-[[3-[(2-méthylquinolin-8-yl)oxyméthyl]-2,4-dichlorophényl]sulfonyl]-2-(S)-[[4-[[(1,1-diméthyléthoxycarbonyl)amino]-[(1,1-diméthyléthoxycarbonyl)imino]méthyl]pipérazin-1-yl]carbonyl]pyrrolidine

En opérant de façon analogue à la préparation XXIII, au départ du composé obtenu selon l'exemple 2, on obtient le produit attendu avec un rendement de 70 %.
**F = 164-168°C**
**[α]**_{**D**}^{**23**}**= +2° (c = 0,26 ; CH**_{**3**}**OH)**

### PREPARATION XXV

### 1-[[3-[(2-méthylquinolin-8-yl)oxyméthyl]-2,4-dichlorophényl]sulfonyl]-2-(S)-[[4-[[(1,1-diméthyléthoxycarbonyl)amino]-[(1,1-diméthyléthoxycarbonyl)imino]méthyl]-3,5-diméthylpipérazin-1-yl]carbonyl]pyrrolidine

En opérant de façon analogue à la préparation XXIII, au départ du composé obtenu selon l'exemple 1, on obtient le produit attendu avec un rendement de 53 %.

### PREPARATION XXVI

### 1-[[3-[(2,4-diméthylquiolin-8-yl)oxyméthyl]-2,4-dichlorophényl]sulfonyl]-2-(S)-[[4-[[(1,1-diméthyléthoxycarbonyl)amino]-[(1,1-diméthyléthoxycarbonyl)imino]méthyl]pipérazin-1-yl]carbonyl]-4-(R)-hydroxypyrrolidine

En opérant de façon analogue à la préparation XXIII, au départ du composé obtenu selon l'exemple 7, on obtient le produit attendu avec un rendement de 77% après cristallisation dans l'éther isopropylique.
**F = 200°C**
**[α]**_{**D**}^{**21**}**= -138° (c = 0,33 ; CHCl**_{**3**}**)**

### PREPARATION XXVII

### 1-[[3-[(2-méthylquinolin-8-yl)oxyméthyl]-2,4-dichlorophényl]sulfonyl]-N-[2-[[(1,1-diméthyléthoxycarbonyl)amino]-[(1,1-diméthyléthoxycarbonyl)imino]méthylamino]éthyl]-2-(S)-pyrrolidinecarboxamide

En opérant de façon analogue à la préparation XXIII, au départ du composé obtenu selon l'exemple 3, on obtient le produit attendu avec un rendement de 50 %.

### PREPARATION XXVIII

### 1-[[3-[(2,4-diméthylquinolin-8-yl)-oxyméthyl]-2,4-dichlorophényl]sulfonyl]-N-[2-[[(1,1-diméthyléthoxycarbonyl)amino]-[(1,1-diméthyléthoxycarbonyl)imino]méthylamino]éthyl]-2-(S)-pyrrolidinecarboxamide

En opérant de façon analogue à la préparation XXIII, au départ du composé obtenu selon l'exemple 4, on obtient le produit attendu avec un rendement de 51 %.
**F = 114-118°C**
**[α]**_{**D**}^{**23**}**= -24° (c = 0,4 ; CH**_{**3**}**OH)**

### PREPARATION XXIX

### 1-[[3-[(2,4-diméthylquinolin-8-yl)oxyméthyl]-2,4-dichlorophényl]sulfonyl]-N-[3-[[(1,1-diméthyléthoxycarbonyl)amino][(1,1-diméthyléthoxycarbonyl)imino]méthylamino]propyl]-2-(S)-pyrrolidinecarboxamide

En opérant de façon analogue à la préparation XXIII, au départ du composé obtenu selon l'exemple 13, on obtient le produit attendu avec un rendement de 70 %.
**F = 106-110°C**
**[α]**_{**D**}^{**22**}**= -20° (c = 0,33 ; CH**_{**3**}**OH)**

### PREPARATION XXX

### 1-[[3-[(2-méthylquinolin-8-yl)oxyméthyl]-2,4-dichlorophényl]sulfonyl]-N-[3-[[(1,1-diméthyléthoxycarbonyl)amino]-[(1,1-diméthyléthoxycarbonyl)imino]méthylamino]propyl]-2-(S)-pyrrolidinecarboxamide

En opérant de façon analogue à la préparation XXIII, au départ du composé obtenu selon l'exemple 9, on obtient le produit attendu avec un rendement de 30 %.
**F = 116-120°C**
**[α]**_{**D**}^{**23**} **= -17° (c = 0,31 ; CH**_{**3**}**OH)**

### PREPARATION XXXI

### 1-[[3-[(2-méthylquinolin-8-yl)oxyméthyl]-2,4-dichlorophényl]sulfonyl]-N-[2-[[(1,1-diméthyléthoxycarbonyl)amino]-[(1,1-diméthyléthoxycarbonyl)imino]méthylamino]éthyl]-N-méthyl-2(S)-pyrrolidinecarboxamide

En opérant de façon analogue à la préparation XXIII, au départ du composé obtenu selon l'exemple 6, on obtient le produit attendu avec un rendement de 68 %.
**F = 95°C**
**[α]**_{**D**}^{**22**} **= -10,4° (c = 0,45 ; CHCl**_{**3**}**)**

### PREPARATION XXXII

### 1-[[3-[(2-méthylquinolin-8-yl)oxyméthyl]-2,4-dichlorophényl]sulfonyl]-N-[[1-[[(1,1-diméthyléthoxycarbonyl)amino]-[(1,1-diméthyléthoxycarbonyl)imino]méthyl]pipéridin-4-yl]méthyl]-2-(S)-pyrrolidinecarboxamide

En opérant de façon analogue à la préparation XXIII, au départ du composé obtenu selon l'exemple 10, on obtient le produit attendu avec un rendement de 58 %.
**F = 104-108°C**
**[α]**_{**D**}^{**22**}**= -32° (c = 0,30 ; CH**_{**3**}**OH)**

### PREPARATION XXXIII

### 1-[[3-[(2-méthylquinolin-8-yl)oxyméthyl]-2,4-dichlorophényl]sulfonyl]-N-[1-[[(1,1-diméthyléthoxycarbonyl)amino]-[(1,1-diméthyléthoxycarbonyl)imino]méthyl]pipéridin-4-yl]-2-(S)-pyrrolidinecarboxamide

En opérant de façon analogue à la préparation XXIII, au départ du composé obtenu selon l'exemple 8, on obtient le produit attendu avec un rendement de 47,3 %.

### PREPARATION XXXIV

### Acide 1-[[3-[(2-méthylquinolin-8-yl)oxyméthyl]-2,4-dichlorophényl]sulfonyl]-2-(S)-pyrrolidinecarboxylique, [1-[[(1,1-diméthyléthoxycarbonyl)amino]-[(1,1-diméthyléthoxycarbonyl)imino]méthyl]pipéridin-4-yl] ester

En opérant de façon analogue à la préparation XXIII, au départ du composé obtenu selon l'exemple 11, on obtient le produit attendu avec un rendement de 80 %.
**F = 75°C**
**[α]**_{**D**}^{**22**} **= +23° (c = 0,25 ; CH**_{**3**}**OH)**

### PREPARATION XXXV

### Acide 1-[[3-[(2-méthylquinolin-8-yl)oxyméthyl]-2,4-dichlorophényl]sulfonyl]-2-(S)-pyrrolidinecarboxylique, [[1-[[(1,1-diméthyléthoxycarbonyl)amino]-[(1,1-diméthyléthoxycarbonyl)imino]méthyl]pipéridin-4-yl]méthyl] ester

En opérant de façon analogue à la préparation XXIII, au départ du composé obtenu selon l'exemple 12, on obtient le produit attendu avec un rendement de 57 %.
**F = 74°C**
**[α]**_{**D**}^{**22**} **= -19° (c = 0,29 ; CH**_{**3**}**OH)**

### Exemple 14

### 1-[[3-[(2,4-diméthylquinolin-8-yl)oxyméthyl]-2,4-dichlorophényl]sulfonyl]-2-(S)-[[4-(amino-imino-méthyl)pipérazin-1-yl]carbonyl]pyrrolidine, bis-trifluoroacétate

En opérant de façon analogue au procédé d'obtention du composé selon l'exemple 5, au départ du composé de la préparation XXIII, on obtient le produit attendu. Celui-ci est ensuite repris en solution dans de l'eau pure, filtré et le filtrat est lyophilisé. On obtient ainsi le produit attendu pur sous forme d'un solide blanc cotonneux (rendement = 79 %).
**F = 145°C**
**[α]**_{**D**}^{**24**}**= +14° (c = 0,34 ; CH**_{**3**}**OH)**

### Exemple 15

### 1-[[3-[(2,4-diméthylquinolin-8-yl)oxyméthyl]-2,4-dichlorophényl]sulfonyl]-2-(S)-[[4-(amino-imino-méthyl)pipérazin-1-yl]carbonyl]-4-(R)-hydroxy-pyrrolidine, bistrifluoroacétate

En opérant de façon analogue au procédé de l'exemple 14, au départ du composé obtenu selon la préparation XXVI, on obtient le produit attendu avec un rendement de 87 %.
**F = 165°C**
**[α]**_{**D**}^{**21**}**= +39° (c = 0,31 ; CH**_{**3**}**OH)**

### Exemple 16

### 1-[[3-[(2-méthylquinolin-8-yl)oxyméthyl]-2,4-dichlorophényl]sulfonyl]-N-[2-[(amino-imino-méthyl)amino]éthyl]-2-(S)-pyrrolidinecarboxamide, bis-trifluoroacétate

En opérant de façon analogue à l'exemple 14, au départ du composé de la préparation XXVII, on obtient le produit attendu avec un rendement de 52 %.
**F = 138-142°C**
**[α]**_{**D**}^{**23**}**= -16° (c = 0,30 ; CH**_{**3**}**OH)**

### Exemple 17

### 1-[[3-[(2-méthylquinolin-8-yl)oxyméthyl]-2,4-dichlorophényl]sulfonyl]-N-[2-[(amino-imino-méthyl)amino]éthyl]-N-(méthyl)-2-(S)-pyrrolidinecarboxamide, bistrifluoroacétate

En opérant de façon analogue à l'exemple 14, au départ du composé de la préparation XXXI, on obtient le produit attendu avec un rendement de 88 %.
**F = 137°C**
**[α]**_{**D**}^{**22**}**= +10,6° (c = 0,48 ; CH**_{**3**}**OH)**

### Exemple 18

### Acide 1-[[3-[(2-méthylquinolin-8-yl)oxyméthyl]-2,4-dichlorophényl]sulfonyl]-2-(S)-pyrrolidinecarboxylique, [[1-(amino-imino-méthyl)pipéridin-4-yl]méthyl] ester, bistrifluoroacétate

En opérant de façon analogue à l'exemple 14, au départ du composé obtenu selon la préparation XXXV, on obtient le produit attendu avec un rendement de 70 %.
**F = 126-130°C**
**[α]**_{**D**}^{**22**} **= -1° (c = 0,38 ; CH**_{**3**}**OH)**

### Exemple 19

### 1-[[3-[(2,4-diméthylquinolin-8-yl)oxyméthyl]-2,4-dichlorophényl]sulfonyl]-N-[2-[(amino-imino-méthyl)amino]éthyl]-2-(S)-pyrrolidinecarboxamide, dichlorhydrate

En opérant de façon analogue au procédé d'obtention du composé selon l'exemple 8, au départ du composé obtenu selon la préparation XXVIII, on obtient le produit attendu avec un rendement de 60 %.
**F = 170-174°C**
**[α]**_{**D**}^{**24**} **= -39° (c = 0,30 ; CH**_{**3**}**OH)**

### Exemple 20

### 1-[[3-[(2,4-diméthylquinolin-8-yl)oxyméthyl]-2,4-dichlorophényl]sulfonyl]-N-[3-[(amino-imino-méthyl)amino]propyl]-2-(S)-pyrrolidinecarboxamide, dichlorhydrate

En opérant de façon analogue à l'exemple 19, au départ du composé obtenu selon la préparation XXIX, on obtient le produit attendu avec un rendement de 80 %.
**F = 170-174°C**
**[α]**_{**D**}^{**24**}**= -34° (c = 0,29 ; CH**_{**3**}**OH)**

### Exemple 21

### 1-[[3-[(2-méthylquinolin-8-yl)oxyméthyl]-2,4-dichlorophényl]sulfonyl]-N-[3-[(amino-imino-méthyl)amino]propyl]-2-(S)-pyrrolidinecarboxamide, dichlorhydrate

En opérant de façon analogue à l'exemple 19, au départ du composé obtenu selon la préparation XXX, on obtient le produit attendu avec un rendement de 52 %.
**F = 158-160°C**
**[α]**_{**D**}^{**22**} **= -30° (c = 0,31 ; CH**_{**3**}**OH)**

### Exemple 22

### 1-[[3-[(2-méthylquinolin-8-yl)oxyméthyl]-2,4-dichlorophényl]sulfonyl]-N-[[1-(amino-imino-méthyl)pipéridin-4-yl]méthyl]-2-(S)-pyrrolidinecarboxamide, dichlorhydrate

En opérant de façon analogue à l'exemple 19, au départ du composé obtenu selon la préparation XXXII, on obtient le produit attendu avec un rendement de 95 %.
**F = 190-192°C**
**[α]**_{**D**}^{**22**}**= -27° (c = 0,35 ; CH**_{**3**}**OH)**

### Exemple 23

### 1-[[3-[(2-méthylquinolin-8-yl)oxyméthyl]-2,4-dichlorophényl]sulfonyl]-N-[1-(amino-imino-méthyl)pipéridin-4-yl]-2-(S)-pyrrolidinecarboxamide, dichlorhydrate

En opérant de façon analogue à l'exemple 19, au départ du composé obtenu selon la préparation XXXIII, on obtient le produit attendu avec un rendement de 47 %.
**F = 152-156°C**
**[α]**_{**D**}^{**22**}**= -17° (c = 0,30 ; CH**_{**3**}**OH)**

### Exemple 24

### 1-[[3-[(2-méthylquinolin-8-yl)oxyméthyl]-2,4-dichlorophényl]sulfonyl]-2-(S)-[[4-(amino-imino-méthyl)pipérazin-1-yl]carbonyl]pyrrolidine, dichlorhydrate

En opérant de façon analogue à l'exemple 19, au départ du composé obtenu selon la préparation XXIV, on obtient le produit attendu avec un rendement de 60 %.
**F = 176-180°C**
**[α]**_{**D**}^{**25**}**= +19° (c = 0,28 ; CH**_{**3**}**OH)**

### Exemple 25

### 1-[[3-[(2-méthylquinolin-8-yl)oxyméthyl]-2,4-dichlorophényl]sulfonyl]-2-(S)-[[4-(amino-imino-méthyl)-3,5-diméthylpipérazin-1-yl]carbonyl]pyrrolidine, dichlorhydrate

En opérant de façon analogue à l'exemple 19, au départ du composé obtenu selon la préparation XXV, on obtient le produit attendu avec un rendement de 70 %.
**F = 186-190°C**
**[α]**_{**D**}^{**22**}**= +21° (c = 0,35 ; CH**_{**3**}**OH)**

### Exemple 26

### Acide 1-[[3-[(2-méthylquinolin-8-yl)oxyméthyl]-2,4-dichlorophényl]sulfonyl]-2-(S)-pyrrolidinecarboxylique, [1-(amino-imino-méthyl)pipéridin-4-yl] ester, dichlorhydrate

En opérant de façon analogue à l'exemple 19, au départ du composé obtenu selon la préparation XXXIV, on obtient le produit attendu avec un rendement de 96 %.
**F = 180-184°C**
**[α]**_{**D**}^{**22**}**= -11° (c = 0,30 ; CH**_{**3**}**OH)**

### Exemple 27

### 1-[[3-[(2-méthylquinolin-8-yl)oxyméthyl]-2,4-dichlorophényl]sulfonyl]-2-(S)-[[4-[(1-méthyléthylamino)(1-méthyléthylimino)méthyl]pipérazin-1-yl]carbonyl]pyrrolidine, dichlorhydrate

a) On prépare une solution de 0,24g (0,426.10⁻³ mole) du composé obtenu selon l'exemple 2 dans 5 ml de méthanol et on ajoute doucement 0,18 ml d'une solution environ 5M de chlorure d'hydrogène dans le méthanol. On laisse agiter pendant 1 heure à température ambiante puis on chasse le solvant sous pression réduite. Le résidu est trituré en présence d'éther éthylique et les cristaux obtenus sont filtrés et séchés. On obtient ainsi 0,24g du chlorhydrate du produit de départ, sous forme de cristaux jaunes.
b) On prépare une suspension de 0,24 g (0,38.10⁻³ mole) du composé obtenu ci-dessus dans 15 ml d'acétonitrile, on ajoute 0,24 g (1,9.10⁻³ mole) de diisopropylcarbodiimide et on agite le mélange à température ambiante pendant 24 heures. Le solvant est ensuite évaporé sous pression réduite et le résidu est lavé plusieurs fois avec de l'acétate d'éthyle chaud. Après séchage des cristaux sous vide, on obtient 90 mg du produit attendu sous forme de cristaux blancs (Rendement = 31 %).
   **F = 148-150°C**
   **[α]**_{**D**}^{**22**}**= -1° (c = 0,30 ; CH**_{**3**}**OH)**

### Exemple 28

### 1-[[3-[(2-méthylquinolin-8-yl)oxyméthyl]-2,4-dichlorophényl]sulfonyl]-2-(S)-[[4-[(cyclohexylamino)(cyclohexylimino)méthyl]pipérazin-1-yl]carbonyl]pyrrolidine, dichlorhydrate

En opérant de façon analogue au procédé de l'exemple 27, au départ de dicyclohexylcarbodiimide, on obtient le produit attendu avec un rendement de 26 %.
**F = 174-178°C**
**[α]**_{**D**}^{**22**}**= +9° (c = 0,28 ; CH**_{**3**}**OH)**

### PREPARATION XXXVI

### 1-[[3-[(2-méthylquinolin-8-yl)oxyméthyl]-2,4-dichlorophényl]sulfonyl]-N-[4-(1,1-diméthyléthoxycarbonylamino)butyl]-2(S)-pyrrolidinecarboxamide

En opérant de façon analogue à la préparation XIV, au départ de N-(1,1-diméthyléthoxycarbonyl)-1,4-butanediamine, on obtient le produit attendu avec un rendement de 70 %.
**F = 72-74°C**
**[α]**_{**D**}^{**22**} **= -48°(c = 0,29 ; CHCl**_{**3**}**)**

### PREPARATION XXXVII

### 1-[[3-[(2-méthylquinolin-8-yl)oxyméthyl]-2,4-dichlorophényl]sulfonyl]-N-[2-[1-(1,1-diméthyléthoxycarbonyl)pipéridin-4-yl]éthyl]-2(S)-pyrrolidinecarboxamide

En opérant de façon analogue à la préparation XIV, au départ de 1-(1,1-diméthyléthoxycarbonyl)-4-(2-aminoéthyl)pipéridine, on obtient le produit attendu avec un rendement de 60 %.
**F = 105°C**
**[α]**_{**D**}^{**22**} **= -35° (c = 0,97 ; CH**_{**3**}**OH)**

### PREPARATION XXXVIII

### 1-[[3-[(2,4-diméthylquinolin-8-yl)oxyméthyl]-2,4-dichlorophényl]sulfonyl]-N-[[1-(1,1-diméthyléthoxycarbonyl)pipéridin-4-yl]méthyl]-2-(S)-pyrrolidinecarboxamide

En opérant de façon analogue à la préparation XIV, au départ de l'acide obtenu selon la préparation XII et de 1-(1,1-diméthyléthoxycarbonyl)-4-(aminométhyl)pipéridine, on obtient le produit attendu avec un rendement de 60%.
**F = 80°C**
**[α]**_{**D**}^{**26**} **= -37° (c = 1,05 ; CH**_{**3**}**OH)**

### PREPARATION XXXIX

### 1-[[3-[(2,4-diméthylquinolin-8-yl)oxyméthyl]-2,4-dichlorophényl]sulfonyl]-N-[1-(1,1-diméthyléthoxycarbonyl)pipéridin-4-yl]-2-(S)-pyrrolidinecarboxamide

En opérant de façon analogue à la préparation XXXVIII, au départ de 1-(1,1-diméthyléthoxycarbonyl)-4-aminopipéridine, on obtient le produit attendu avec un rendement de 54 %.
**F = 60-64°C**
**[α]**_{**D**}^{**25**} **= -21° (c = 1,32 ; CH**_{**3**}**OH)**

### Exemple 29 (exemple préparatif)

### 1-[[3-[(2-méthylquinolin-8-yl)oxyméthyl]-2,4-dichlorophényl]sulfonyl]-N-(4-aminobutyl)-2-(S)-pyrrolidinecarboxamide, dichlorhydrate

On ajoute 2,7 g (4.10⁻³ mole) du composé obtenu selon la préparation XXXVI dans 250 ml d'acide chlorhydrique 1N et on agite le mélange à 45 °C pendant 2 heures. On concentre ensuite sous pression réduite puis on dilue avec 50 ml d'eau. La solution ainsi obtenue est filtrée et lyophilisée. On obtient ainsi le produit attendu sous forme d'un solide blanc avec un rendement de 95 %.
**F = 164-166°C**
**[α]**_{**D**}^{**22**}**= -28° (c = 0,3 ; CH**_{**3**}**OH)**

### Exemple 30

### 1-[[3-[(2-méthylquinolin-8-yl)oxyméthyl] -2,4-dichlorophényl] sulfonyl]-N-[2-(pipéridin-4-yl)éthyl]-2-(S)-pyrrolidinecarboxamide, dichlorhydrate

En opérant de façon analogue à l'exemple 8, au départ du composé obtenu selon la préparation XXXVII, on obtient le produit attendu avec un rendement de 98 %.
**F = 174°C**
**[α]**_{**D**}^{**22**}**= - 30° (c =1,0 ; CH**_{**3**}**OH)**

### Exemple 31

### 1-[[3-[(2,4-diméthylquinolin-8-yl)oxyméthyl]-2,4-dichlorophényl]sulfonyl]-N-[(pipéridin-4-yl)méthyl]-2-(S)-pyrrolidinecarboxamide, dichlorhydrate

En opérant de façon analogue à l'exemple 8, au départ du composé obtenu selon la préparation XXXVIII, on obtient le produit attendu avec un rendement de 97 %.
**F = 195 °C**
**[α]**_{**D**}^{**24**}**= - 32° (c =1,0 ; CH**_{**3**}**OH)**

### Exemple 32

### 1-[[3-[(2,4-diméthylquinolin-8-yl)oxyméthyl]-2,4-dichlorophényl]sulfonyl]-N-[pipéridin-4-yl]-2-(S)-pyrrolidinecarboxamide, dichlorhydrate

En opérant de façon analogue à l'exemple 8, au départ du composé obtenu selon la préparation IXL, on obtient le produit attendu avec un rendement de 85 %.
**F = 184-190°C**
**[α]**_{**D**}^{**25**}**= -14° (c = 0,56 ; CH**_{**3**}**OH)**

### PREPARATION XL

### 1-[[3-[(2-méthylquinolin-8-yl)oxynléthyl]-2,4-dichlorophényl]sulfonyl]-N-[4-[[(1,1-diméthyléthoxycarbonyl)amino]-[(1,1-diméthyléthoxycarbonyl)imino]méthylamino]butyl)-2-(S)-pyrrolidinecarboxamide

En opérant de façon analogue à la préparation XXIII, au départ du composé obtenu selon l'exemple 29, on obtient le produit attendu avec un rendement de 50 %.
**F = 82-84°C**
**[α]**_{**D**}^{**22**}**= -37° (c = 0,31 ; CHCl**_{**3**}**)**

### PREPARATION XLI

### 1-[[3-[(2-méthylquinolin-8-yl)oxyméthyl]-2,4-dichlorophényl]sulfonyl]-N-[2-[1-[[(1,1-diméthyléthoxycarbonyl)amino]-[(1,1-diméthyléthoxycarbonyl)imino]méthyl]pipéridin-4-yl]éthyl]-2-(S)-pyrrolidinecarboxamide

En opérant de façon analogue à la préparation XXIII, au départ du composé obtenu selon l'exemple 30, on obtient le produit attendu avec un rendement de 79 %.
**F = 80°C**
**[α]**_{**D**}^{**22**}**= -36° (c = 1 ; CH**_{**3**}**OH)**

### PREPARATION XLII

### 1-[[3-[(2,4-diméthylquinolin-8-yl)oxyméthyl]-2,4-dichlorophényl]sulfonyl]-N-[[1-[[(1,1-diméthyléthoxycarbonyl)amino]-[(1,1-diméthyléthoxycarbonyl)imino]méthyl]pipéridin-4-yl]méthyl]-2-(S)-pyrrolidinecarboxamide

En opérant de façon analogue à la préparation XXIII, au départ du composé obtenu selon l'exemple 31, on obtient le produit attendu avec un rendement de 75 %.
**F = 98°C**
**[α]**_{**D**}^{**22**} **= -31° (c = 1,05 ; CH**_{**3**}**OH)**

### PREPARATION XLIII

### 1-[[3-[(2-méthylquinolin-8-yl)oxyméthyl]-2,4-dichlorophényl]sulfonyl]-2-(S)-[[4-[[(éthyl)-(1,1-diméthyléthoxycarbonyl)amino]-[(1,1-diméthyléthoxycarbonyl)imino]méthyl]pipérazin-1-yl]carbonyl]pyrrolidine

A une solution de 0,15 g (0,186.10⁻³ mole) du composé selon la préparation XXIV dans 5 ml de diméthylformamide, on ajoute 8 mg (0,186.10⁻³ mole) d'hydrure de sodium en suspension à 60 % dans l'huile. Après 5 mn sous agitation à température ambiante, on ajoute 58 mg (0,372.10⁻³ mole) d'iodure d'éthyle. Le mélange est agité pendant 20 heures à température ambiante puis versé sur 100 ml d'eau. Le précipité formé est filtré, rincé à l'eau et séché en étuve à vide à 50 °C. On obtient ainsi 0,14 g du produit attendu sous forme d'un solide blanc (rendement = 90 %).
**F = 95 °C**
**[α]**_{**D**}^{**23**} **= - 36° (c = 0,32 ; CH**_{**3**}**OH)**

### Exemple 33

### 1-[[3-[(2-méthylquinolin-8-yl)oxyméthyl]-2,4-dichlorophényl]sulfonyl]-N-[4-[(amino-imino-méthyl)amino]butyl]-2-(S)-pyrrolidinecarboxamide, dichlorhydrate

En opérant de façon analogue à l'exemple 29, au départ du composé obtenu selon la préparation XL, on obtient le produit attendu avec un rendement de 70 %.
**F = 148-152°C**
**[α]**_{**D**}^{**24**}**= -31° (c = 0,35 ; CH**_{**3**}**OH)**

### Exemple 34

### 1-[[3-[(2-méthylquinolin-8-yl)oxyméthyl]-2,4-dichlorophényl]sulfonyl]-N-[2-[1-(amino-imino-méthyl)pipéridin-4-yl]éthyl]-2-(S)-pyrrolidinecarboxamide, dichlorhydrate

En opérant de façon analogue à l'exemple 8, au départ du composé obtenu selon la préparation XLI, on obtient le produit attendu avec un rendement de 82 %.
**F = 165°C**
**[α]**_{**D**}^{**22**}**= -24,5° (c = 1,05 ; CH**_{**3**}**OH)**

### Exemple 35

### 1-[[3-[(2,4-diméthylquinolin-8-yl)oxyméthyl]-2,4-dichlorophényl]sulfonyl]-N-[[1-(amino-imino-méthyl)pipéridin-4-yl]méthyl]-2-(S)-pyrrolidinecarboxamide, dichlorhydrate

En opérant de façon analogue à l'exemple 8, au départ du composé obtenu selon la préparation XLII, on obtient le produit attendu avec un rendement de 76 %.
**F = 211°C**
**[α]**_{**D**}^{**22**} **= -26,5° (c = 1,0 ; CH**_{**3**}**OH)**

### Exemple 36

### 1-[[3-[(2-méthylquinolin-8-yl)oxyméthyl]-2,4-dichlorophényl]sulfonyl]-2-(S)-[[4-[(éthylamino)-imino-méthyl]pipérazin-1-yl]carbonyl]pyrrolidine, bistrifluoroacétate

On prépare une solution de 0,34 g (0,408.10⁻³ mole) du composé selon la préparation XLIII dans 15 ml de dichlorométhane, puis on ajoute 88 mg (0,816.10⁻³ mole) d'anisole, on refroidit à 0°C et on ajoute 3 ml d'acide trifluoroacétique. On agite à 0 °C pendant une heure puis pendant une nuit à température ambiante. Les solvants sont évaporés sous pression réduite et le résidu est repris dans de l'éther isopropylique. Le précipité formé est filtré et séché puis repris en solution dans 5 ml d'eau et lyophilisé. On obtient ainsi 0,23 g du produit attendu sous forme d'un solide blanc (rendement = 65 %).
**F = 115°C**
**[α]**_{**D**}^{**23**}**= +10° (c = 0,42 ; CH**_{**3**}**OH)**

### PREPARATION XLIV

### 1-[[3-[(2-méthylquinolin-8-yl)oxyméthyl]-2,4-dichlorophényl]sulfonyl]-N-[[1-[4-[(1,1-diméthyléthoxycarbonyl)amino]-1-oxobutyl]pipéridin-4-yl]méthyl]-2-(S)-pyrrolidinecarboxamide

En opérant de façon analogue à la préparation XIV, au départ du composé obtenu selon l'exemple 10 et d'acide 4-[(1,1-diméthyléthoxycarbonyl)amino]butanoïque, en présence de N-méthyl-morpholine, on obtient le produit attendu avec un rendement de 88 %.
**F = 98°C**
**[α]**_{**D**}^{**22**}**= -39° (c = 1,05 ; CH**_{**3**}**OH)**

### PREPARATION XLV

### 1-[[3-[(2-méthylquinolin-8-yl)oxyméthyl]-2,4-dichlorophényl]sulfonyl]-N-[1-[2-[(1,1-diméthyléthoxycarbonyl)amino]-1-oxoéthyl]pipéridin-4-yl]-2-(S)-pyrrolidinecarboxamide

En opérant de façon analogue à la préparation XLIV, au départ du composé selon l'exemple 8 et de N-(1,1-diméthyléthoxycarbonyl)glycine, on obtient le produit attendu avec un rendement de 97 %.
**F = 94°C**
**[α]**_{**D**}^{**22**} **= -40° (c = 1,03 ; CHCl**_{**3**}**)**

### PREPARATION XLVI

### 1-[[3-[(2-méthylquinolin-8-yl)oxyméthyl]-2,4-dichlorophényl]sulfonyl]-N-[1-[3-[(1,1-diméthyléthoxycarbonyl)amino]-1-oxopropyl]pipéridin-4-yl]-2-(S)-pyrrolidinecarboxamide

En opérant de façon analogue à la préparation XLIV, au départ du composé selon l'exemple 8 et de N-(1,1-diméthyléthoxycarbonyl)-β-alanine, on obtient le produit attendu avec un rendement de 97 %.
**F = 100-102°C**
**[α]**_{**D**}^{**22**} **= -39° (c = 1,0; (CHCl**_{**3**}**)**

### PREPARATION XLVII

### 1-[[3-[(2-méthylquinolin-8-yl)oxyméthyl]-2,4-dichlorophényl]sulfonyl]-N-[1-[4-[(1,1-diméthyléthoxycarbonyl)amino]-1-oxobutyl]pipéridin-4-yl]-2-(S)-pyrrolidinecarboxamide

En opérant de façon analogue à la préparation XLIV, au départ du composé selon l'exemple 8 et d'acide 4-[(1,1-diméthyléthoxycarbonyl)amino]butanoïque, on obtient le produit attendu avec un rendement de 91 %.
**F = 98°C**
**[α]**_{**D**}^{**22**} **= -39° (c = 0,95 ; CHCl**_{**3**}**)**

### PREPARATION XLVIII

### 1-[[3-[(2,4-diméthylquinolin-8-yl)oxyméthyl]-2,4-dichlorophényl]sulfonyl]-N-[1-[3-[(1,1-diméthyléthoxycarbonyl)amino]-1-oxopropyl]pipéridin-4-yl]-2-(S)-pyrrolidinecarboxamide

En opérant de façon analogue à la préparation XLVI, au départ du composé selon l'exemple 32 et de N-(1,1-diméthyléthoxycarbonyl)-β-alanine, on obtient le produit attendu avec un rendement de 98 %.
**F = 75-78°C**
**[α]**_{**D**}^{**22**}**= -53° (c = 1,05 ; CH**_{**3**}**OH)**

### Exemple 37

### 1-[[3-[(2-méthylquinolin-8-yl)oxyméthyl]-2,4-dichlorophényl]sulfonyl]-N-[[1-(4-amino-1-oxobutyl)pipéridin-4-yl]méthyl]-2-(S)-pyrrolidinecarboxamide, dichlorhydrate

En opérant de façon analogue à l'exemple 8, au départ du composé obtenu selon la préparation XLIV, on obtient le produit attendu avec un rendement de 94 %.
**F = 151°C**
**[α]**_{**D**}^{**22**} **= -22,5° (c = 0,95 ; CH**_{**3**}**OH)**

### Exemple 38

### 1-[[3-[(2-méthylquinolin-8-yl)oxyméthyl]-2,4-dichlorophényl]sulfonyl]-N-[1-(2-amine-1-oxoéthyl)pipéridin-4-yl]-2-(S)-pyrrolidinecarboxamide, dichlorhydrate

En opérant de façon analogue à l'exemple 8, au départ du composé obtenu selon la préparation XLV, on obtient le produit attendu avec un rendement de 89 %.
**F = 210°C**
**[α]**_{**D**}^{**22**} **= -8,6° (c = 0,95 ; CH**_{**3**}**OH)**

### Exemple 39

### 1-[[3-[(2-méthylquinolin-8-yl)oxyméthyl]-2,4-dichlorophényl]sulfonyl]-N-[1-(3-amino-1-oxopropyl)pipéridin-4-yl]-2-(S)-pyrrolidinecarboxamide, dichlorhydrate

En opérant de façon analogue à l'exemple 8, au départ du composé obtenu selon la préparation XLVI, on obtient le produit attendu avec un rendement de 19 %.
**F = 140°C**
**[α]**_{**D**}^{**22**}**= -33° (c = 0,75 ; CHCl**_{**3**}**)**

### Exemple 40

### 1-[[3-[(2-méthylquinolin-8-yl)oxyméthyl]-2,4-dichlorophényl]sulfonyl]-N-[1-(4-amino-1-oxobutyl)pipéridin-4-yl]-2-(S)-pyrrolidinecarboxamide, dichlorhydrate

En opérant de façon analogue à l'exemple 8, au départ du composé obtenu selon la préparation XLVII, on obtient le produit attendu avec un rendement de 99 %.
**F = 174°C**
**[α]**_{**D**}^{**22**}**= -7,7° (c = 1,0 ; CH**_{**3**}**OH)**

### Exemple 41

### 1-[[3-[(2,4-diméthylquinolin-8-yl)oxyméthyl]-2,4-dichlorophényl]sulfonyl]-N-[1-(3-amino-1-oxopropyl)pipéridin-4-yl]-2-(S)-pyrrolidinecarboxamide, dichlorhydrate

En opérant de façon analogue à l'exemple 8, au départ du composé obtenu selon la préparation XLVIII, on obtient le produit attendu avec un rendement de 98 %.
**F = 89-91°C**
**[α]**_{**D**}^{**22**} **= -12,5° (c = 1,1 ; CH**_{**3**}**OH)**

### PREPARATION IL

### 1-[[3-[(2-méthylquinolin-8-yl)oxyméthyl]-2,4-dichlorophényl]sulfonyl]-N-[[1-[4-[[(1,1-diméthyléthoxycarbonyl)amino]-[(1,1-diméthyléthoxycarbonyl)imino]méthylamino]-1-oxobutyl]pipéridin-4-yl]méthyl]-2-(S)-pyrrolidine carboxamide

On prépare une solution de 1,9 g (2,6.10⁻³ mole) du composé obtenu selon l'exemple 37 dans 40 ml d'éthanol, on ajoute 1,4 ml (10.10⁻³ mole) de triéthylamine puis, après environ 5 mn d'agitation, on ajoute 0,9 g (3,1.10⁻³ mole) de N,N'-bis(1,1-diméthyléthoxycarbonyl)-S-méthylisothiourée {ou acide [[[(1,1-diméthyléthoxy)carbonyl]amino](méthylthio)méthylène]carbamique, 1,1-diméthyléthylester} ; on ajoute enfin 1,8 g (8.10⁻³ mole) d'oxyde mercurique et on laisse sous agitation à température ambiante pendant 18 heures. On filtre le milieu réactionnel puis on concentre le filtrat sous pression réduite. Le résidu est purifié par chromatographie sur gel de silice en éluant avec de l'acétate d'éthyle. On obtient ainsi 1,7 g du produit attendu sous forme de cristaux blancs (rendement = 69 %).
**F = 90°C**
**[α]**_{**D**}^{**20**}**= -32° (c = 1,05 ; CH**_{**3**}**OH)**

### PREPARATION L

### 1-[[3-[(2-méthylquinolin-8-yl)oxyméthyl]-2,4-dichlorophényl]sulfonyl]-N-[1-[2-[[(1,1-diméthyléthoxycarbonyl)amino]-[(1,1-diméthyléthoxycarbonyl)imino]méthylamino]-1-oxoéthyl]pipéridin-4-yl]-2-(S)-pyrrolidinecarboxamide

En opérant de façon analogue à la préparation IL, au départ du composé obtenu selon l'exemple 38, on obtient le produit attendu avec un rendement de 15 %.
**F = 123°C**
**[α]**_{**D**}^{**22**}**= -32,5° (c = 0,9 ; CHCl**_{**3**}**)**

### PREPARATION LI

### 1-[[3-[(2-méthylquinolin-8-yl)oxyméthyl]-2,4-dichlorophényl]sulfonyla-N-[1-[3-[[(1,1-diméthyléthoxycarbonyl)amino]-[(1,1-diméthyléthoxycarbonyl)imino]méthylamino]-1-oxopropyl]pipéridin-4-yl]-2-(S)-pyrrolidinecarboxamide

En opérant de façon analogue à la préparation IL, au départ du composé obtenu selon l'exemple 39, on obtient le produit attendu avec un rendement de 77 %.
**F = 112°C**
**[α]**_{**D**}^{**22**} **= -23° (c = 1,3 ; CH**_{**3**}**OH)**

### PREPARATION LII

### 1-[[3-[(2-méthylquinolin-8-yl)oxyméthyl]-2,4-dichlorophényl]sulfonyl]-N-[1-[4-[[(1,1-diméthyléthoxycarbonyl)amino]-[(1,1-diméthyléthoxycarbonyl)imino]méthylamino]-1-oxobutyl]pipéridin-4-yl]-2-(S)-pyrrolidinecarboxamide

En opérant de façon analogue à la préparation IL, au départ du composé obtenu selon l'exemple 40, on obtient le produit attendu avec un rendement de 74 %.
**F = 102-105°C**
**[α]**_{**D**}^{**20**} **= -24° (c = 1,05 ; CH**_{**3**}**OH)**

### PREPARATION LIII

### 1-[[3-[(2,4-diméthylquinolin-8-yl)oxyméthyl]-2,4-dichlorophényl]sulfonyl]-N-[1-[3-[[(1,1-diméthyléthoxycarbonyl)amino]-[(1,1-diméthyléthoxycarbonyl)imino]méthylamino]-1-oxopropyl]pipéridin-4-yl]-2-(S)-pyrrolidinecarboxamide

En opérant de façon analogue à la préparation IL, au départ du composé obtenu selon l'exemple 41, on obtient le produit attendu avec un rendement de 86 %.
**F = 115-117°C**
**[α]**_{**D**}^{**22**} **= -22° (c = 0,92 ; CH**_{**3**}**OH)**

### Exemple 42

### 1-[[3-[(2-méthylquinolin-8-yl)oxyméthyl]-2,4-dichlorophényl]sulfonyl]-N-[[1-[4-[(amine-imino-méthyl)amino]-1-oxobutyl]pipéridin-4-yl]méthyl]-2-(S)-pyrrolidinecarboxamide, dichlorhydrate

En opérant de façon analogue à l'exemple 8, au départ du composé obtenu selon la préparation IL, on obtient le produit attendu avec un rendement de 75 %.
**F = 178°C**
**[α]**_{**D**}^{**22**}**= -19° (c = 1,05 ; CB**_{**3**}**OH)**

### Exemple 43

### 1-[[3-[(2-méthylquinolin-8-yl)oxyméthyl]-2,4-dichlorophényl]sulfonyl]-N-[1-[2-[(amino-imino-méthyl)amino]-1-oxoéthyl]pipéridin-4-yl]-2-(S)-pyrrolidinecarboxamide, dichlorhydrate

En opérant de façon analogue à l'exemple 8, au départ du composé obtenu selon la préparation L, on obtient le produit attendu avec un rendement de 83 %.
**F = 194°C**
**[α]**_{**D**}^{**22**}**= -8,7° (c = 0,65 ; CH**_{**3**}**OH)**

### Exemple 44

### 1-[[3-[(2-méthylquinolin-8-yl)oxyméthyl]-2,4-dichlorophényl]sulfonyl]-N-[1-[3-[(amino-imino-méthyl)amino]-1-oxopropyl]pipéridin-4-yl]-2-(S)-pyrrolidinecarboxamide, dichlorhydrate

En opérant de façon analogue à l'exemple 8, au départ du composé obtenu selon la préparation LI, on obtient le produit attendu avec un rendement de 81 %.
**F = 170°C**
**[α]**_{**D**}^{**22**}**= -11,5° (c = 1,1 ; CH**_{**3**}**OH)**

### Exemple 45

### 1-[[3-[(2-méthylquinolin-8-yl)oxyméthyl]-2,4-dichlorophényl]sulfonyl]-N-[1-[4-[(amino-imino-méthyl)amino]-1-oxobutyl]pipéridin-4-yl]-2-(S)-pyrrolidinecarboxamide, dichlorhydrate

En opérant de façon analogue à l'exemple 8, au départ du composé obtenu selon la préparation LII, on obtient le produit attendu avec un rendement de 85 %.
**F = 190°C**
**[α]**_{**D**}^{**22**}**= -6,1° (c = 1,05 ; CH**_{**3**}**OH)**

### Exemple 46

### 1-[[3-[(2,4-diméthylquinolin-8-yl)oxyméthyl]-2,4-dichlorophényl]sulfonyl]-N-[1-[3-[(amino-imino-méthyl)amino]-1-oxopropyl]pipéridin-4-yl]-2-(S)-pyrrolidinecarboxamide, dichlorhydrate

En opérant de façon analogue à l'exemple 8, au départ du composé obtenu selon la préparation LIII, on obtient le produit attendu avec un rendement de 74 %.
**F = 174°C**
**[α]**_{**D**}^{**22**} **= -9,6° (c = 1,0 ; CH**_{**3**}**OH)**

L'activité des produits selon l'invention a été appréciée en fonction de leur aptitude à se lier aux récepteurs de la bradykinine. En effet, les kinines, dont le principal représentant est la bradykinine, forment un groupe de petits peptides qui contribuent de façon importante à la réponse inflammatoire et apparaissent de ce fait impliqués dans la pathophysiologie des maladies inflammatoires. De plus, la bradykinine est un des agents algésiants parmi les plus puissants connus. Les kinines activent deux types de récepteurs appelés respectivement B₁ et B₂ qui appartiennent à la grande famille des récepteurs à sept domaines transmembranaires couplés aux G-protéines. On décrit dans la présente invention des composés qui se lient au récepteur B₂ et bloquent de ce fait la fixation de la bradykinine à ce récepteur.

Le test pharmacologique utilisé est le suivant : des segments d'iléon de cobayes mâles de souche Dunkin-Hartley (Iffa Credo, l'Arbresle, France) sont isolés et broyés dans le tampon TES suivant : TES 25mM, 1,10-phénanthroline 1mM (pH 6.8), bacitracine 140 *µ*g/ml, BSA 1g/l. Les membranes sont ensuite isolées par centrifugation (18000 tours par minute ; 20 min ; 4 °C). Les études de liaison sont effectuées dans le tampon TES en utilisant la [³H]-bradykinine (120 pM), 50 *µ*g de protéine membranaire par essai (volume final 500 *µ*l) avec un temps d'équilibre de 90 min à 20°C. On détermine ensuite le taux (en pourcentage) d'inhibition de la fixation de [³H]-bradykinine en présence de l'un des composés selon l'invention à tester à une concentration de 10⁻⁶ M.

Les résultats obtenus (notés "activité") lors de ces essais sont consignés dans le tableau I ci-après en regard des exemples figurant dans la description.

Les composés de la présente invention qui inhibent la liaison de la [³H]-bradykinine au récepteur B₂ de cobaye (voir tableau I) se lient également au récepteur B₂ humain cloné et transfecté de façon stable dans des cellules CHO (Chinese Hamster Ovary cells). Ainsi dans ce test, certains composés à la concentration de 10 *µ*M inhibent d'au moins 95 % la fixation de la [³H]-bradykinine au récepteur B₂.

Les composés de la présente invention sont utiles dans le traitement des algies, et en particulier dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastrointestinal (par ex. colites, rectites, maladie de Crohn), la pancréatite, certains carcinomes, l'angiooedème héréditaire, la migraine, l'encéphalomyélite, la méningite, les accidents vasculaires cérébraux (notamment ceux provoqués par un choc traumatique cérébral), certains désordres neurologiques, les états inflammatoires vasculaires (par exemple : athérosclérose et artérite des membres inférieurs), les états douloureux (par exemple les céphalgies, les douleurs dentaires, les douleurs menstruelles), les contractions utérines prématurées, la cystite et les brûlures. Les composés selon l'invention peuvent également être utiles pour la potentialisation d'agents antiviraux.

Les composés de la présente invention, qui peuvent être utilisés sous forme de base libre ou de leurs sels d'addition non-toxiques, en association avec un excipient physiologiquement acceptable, seront en général prescrits en thérapeutique à des doses d'environ 1 à 1000 mg/jour, sous une forme administrable par voie orale, par injection intraveineuse, intramusculaire ou sous-cutanée, par voie transdermique, par le moyen d'aérosols ou par le moyen de suppositoires.

Les composés pourront également être administrés par voie topique, par exemple sous forme de gels ou de pommades.

Les composés de la présente invention trouvent également leur utilité, en tant que réactifs pharmacologiques, notamment pour l'étude des interactions hormone-récepteur. L'utilisation en tant que réactif pharmacologique peut faire appel à un dérivé radiomarqué de l'un des composés selon l'invention (par exemple avec du tritium [³H] ou du soufre [³⁵S]), dans le but d'obtenir un radio-ligand destiné à des études conformationnelles du récepteur B₂ de la bradykinine, ou des tests de fixation à ce type de récepteur, par exemple pour l'évaluation de nouveaux composés susceptibles de présenter une affinité pour le récepteur B₂ de la bradykinine.

Selon l'invention, l'on préconise une composition thérapeutique qui est caractérisée en ce qu'elle renferme, en association avec un excipient physiologiquement acceptable, au moins un ingrédient actif choisi parmi l'ensemble constitué par les composés de formule I et leurs sels d'addition non-toxiques.

L'invention vise en outre des composés intermédiaires utiles dans la synthèse intermédiaire pour la synthèse des composés de formule I dans laquelle R₃ est le groupe 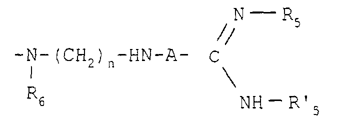 où A, R₅ et R'₅ sont définis comme dans la formule I de ladite revendication 1, n est 2, 3 ou 4 et R₆ est H ou alkyle en C₁-C₃.

Ces composés intermédiaires (i.e. exemples préparatifs 3, 4, 6, 9, 13 et 29) sont caractérisés en ce qu'ils sont choisis parmi l'ensemble constitué par les :
1-[[3-[(2-méthylquinolin-8-yl)oxyméthyl]-2,4-dichlorophényl]sulfonyl]-N-(2-aminoéthyl)-2-(S)-pyrrolidinecarboxamide,
1-[[3-[(2,4-diméthylquinolin-8-yl)oxyméthyl]-2,4-dichlorophényl]sulfonyl]-N-(2-aminoéthyl)-2-(S)-pyrrolidinecarboxamide,
1-[[3 - [(2-méthylquinolin-8-yl)oxyméthyl] -2,4-dichlorophényl]sulfonyl]-N-méthyl-N-(2-aminoéthyl)-2-(S)-pyrrolidinecarboxamide,
1-[[3-[(2-méthylquinolin-8-yl)oxyméthyl]-2,4-dichlorophényl]sulfonyl]-N-(4-aminobutyl)-2-(S)-pyrrolidinecarboxamide,
1-[[3-[(2,4-diméthylquinolin-8-yl)oxyméthyl]-2,4-dichlorophényl]sulfonyl]-N-(3-aminopropyl)-2-(S)-pyrrolidinecarboxamide, et
1-[[3-[(2-méthylquinolin-8-yl)oxyméthyl]-2,4-dichlorophényl]sulfonyl]-N-(4-aminobutyl)-2-(S)-pyrrolidinecarboxamide.

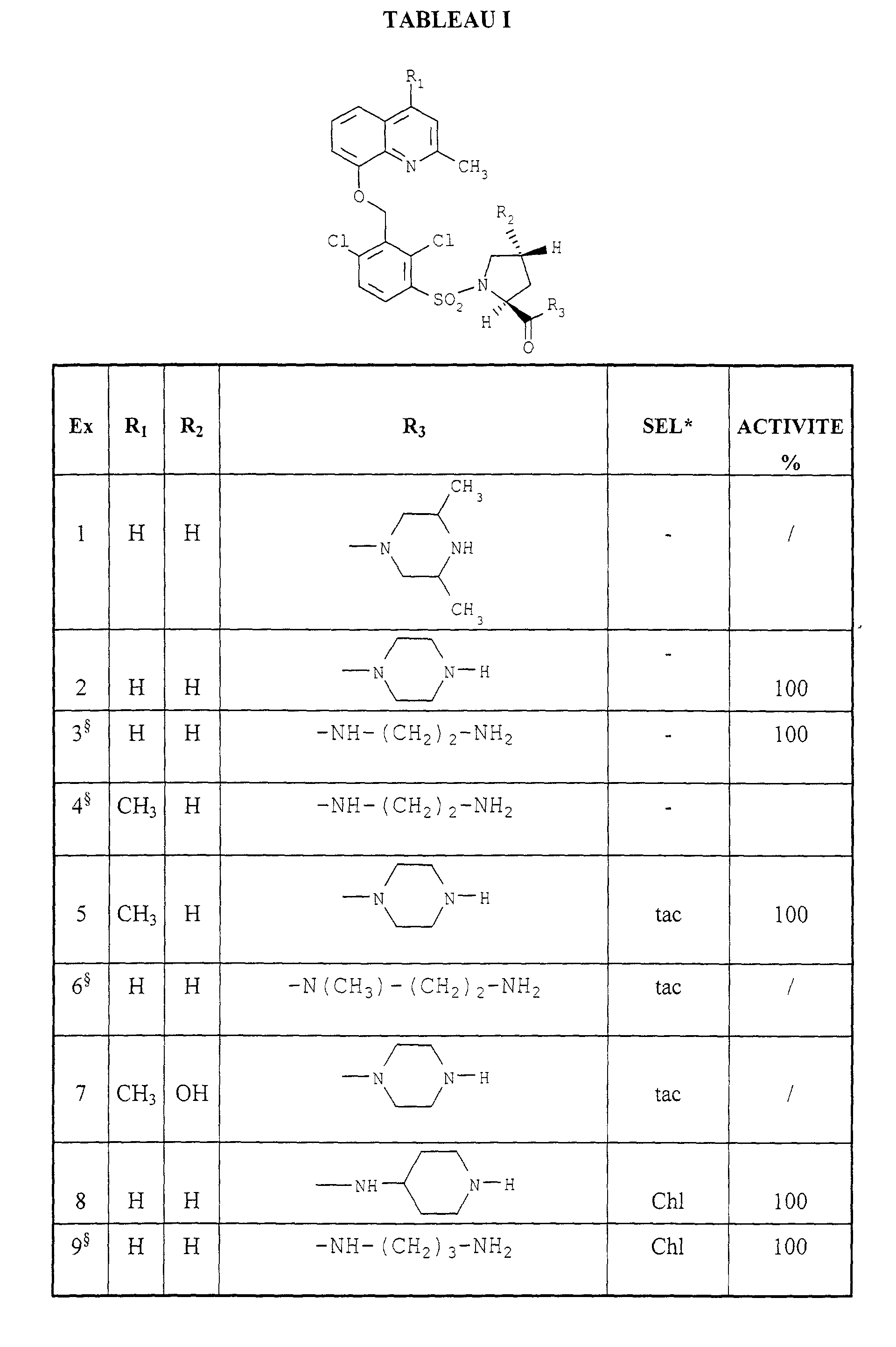

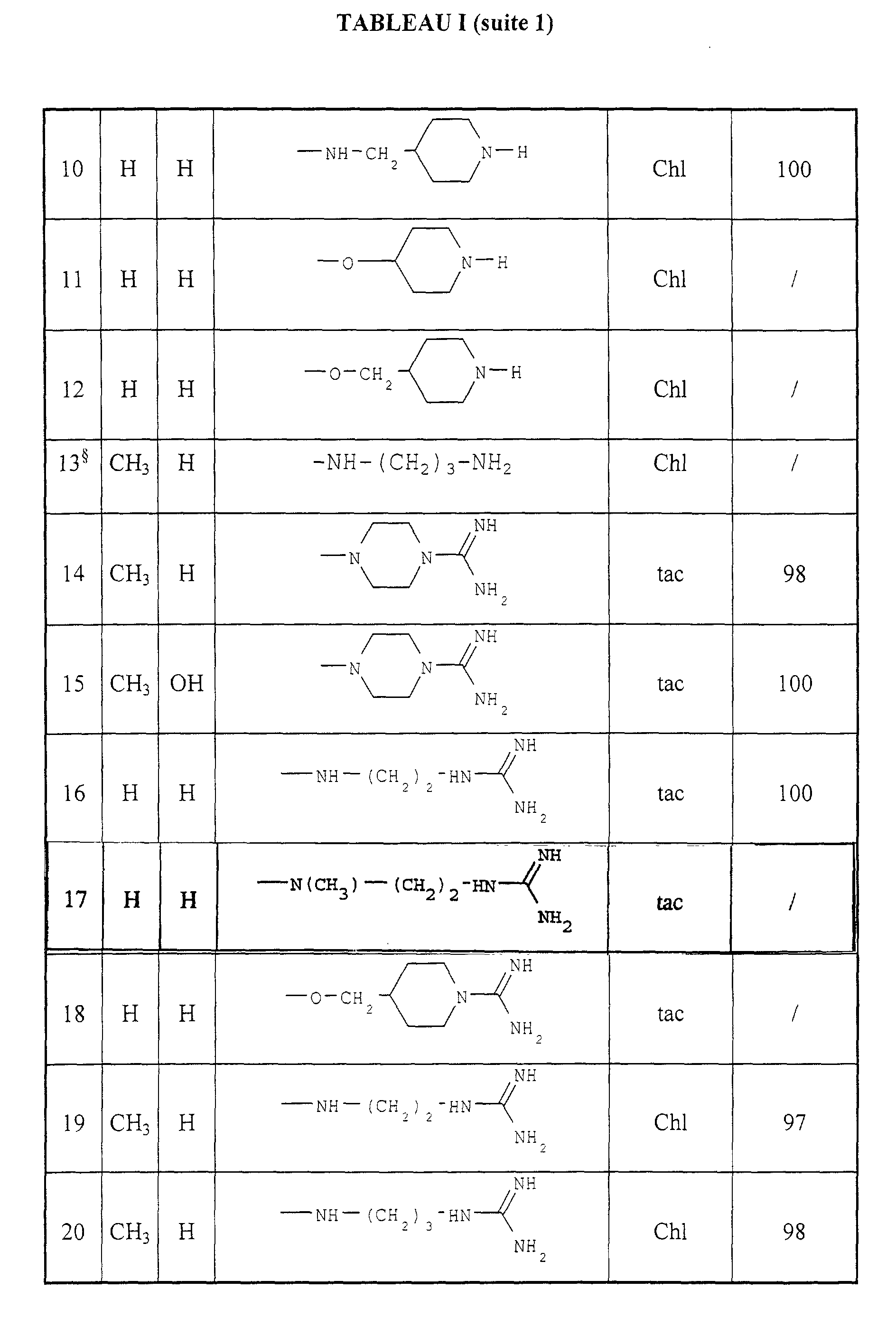

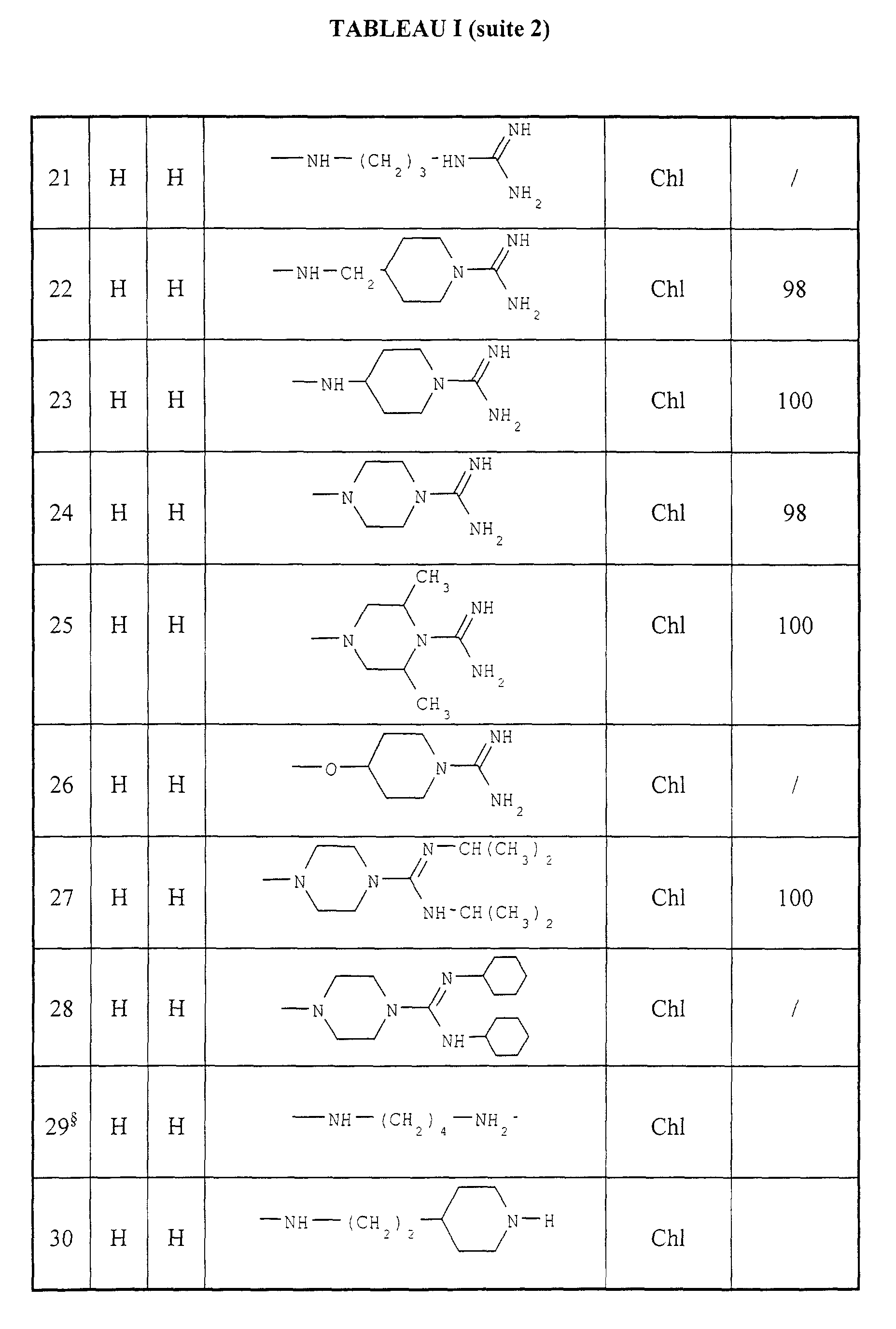

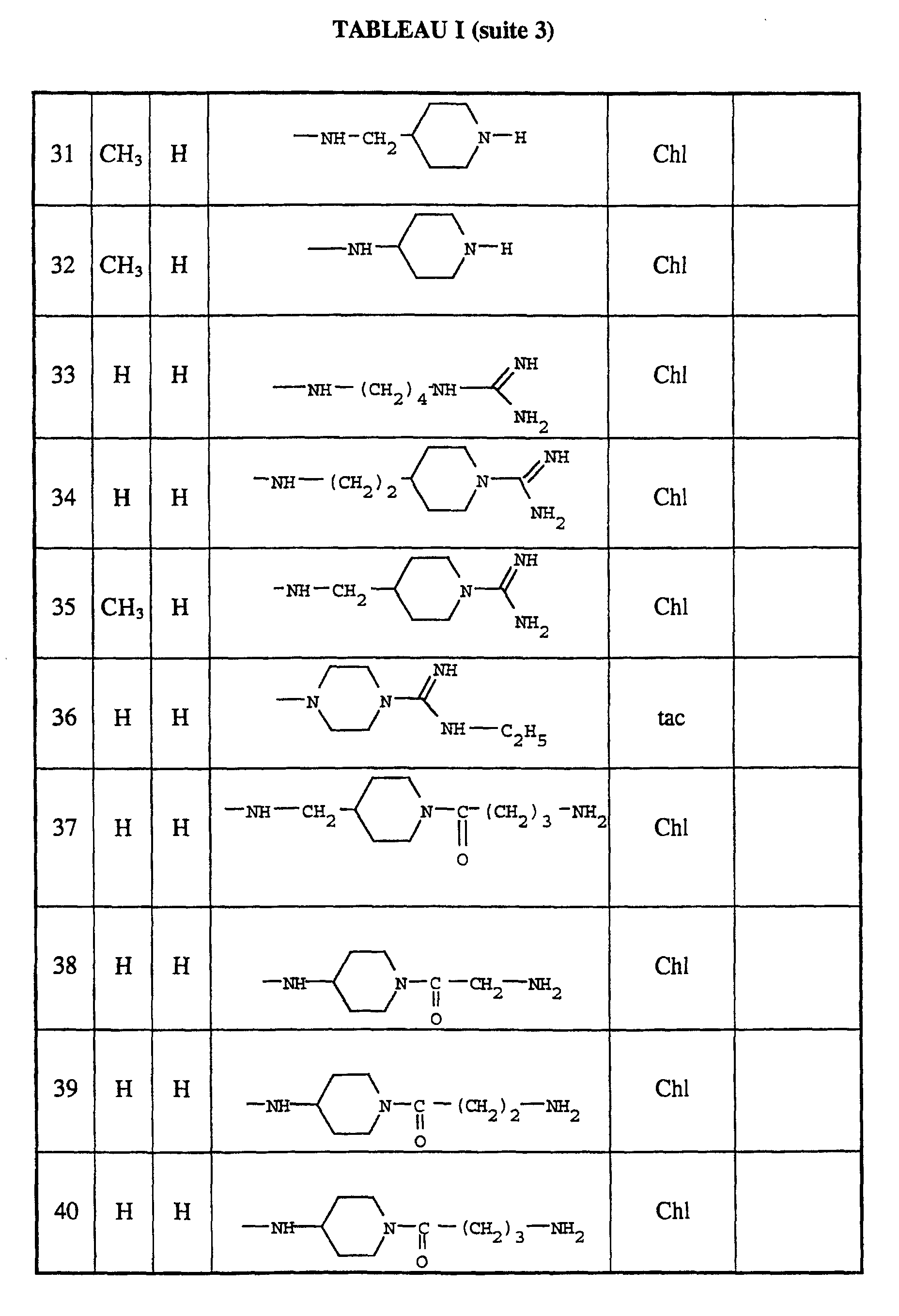

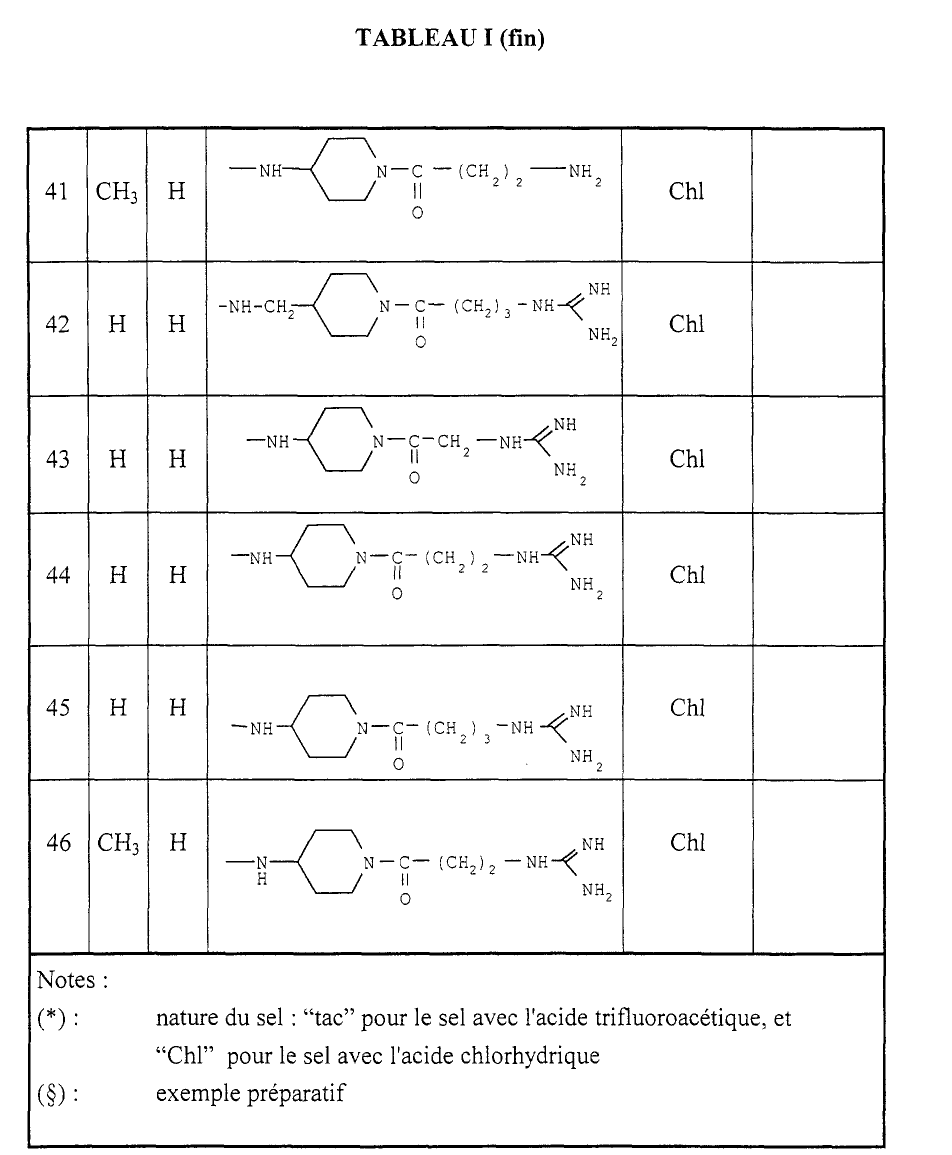

## Revendications

1. Composé de N-benzènesulfonylpyrrolidine, **caractérisé en ce qu'**il est choisi parmi l'ensemble constitué par
(i) les composés de formule : 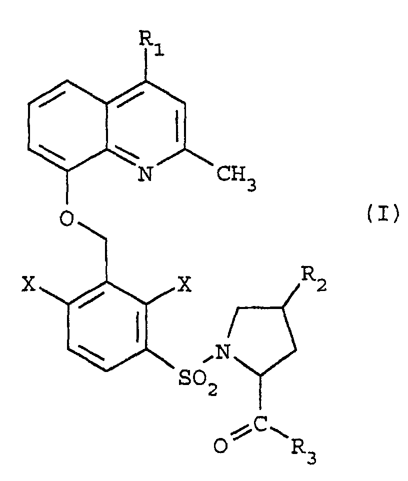 dans laquelle :
X représente un atome d'halogène,
R₁ représente un atome d'hydrogène ou un groupe alkyle en C₁-C₃ à chaîne hydrocarbonnée linéaire ou ramifiée,
R₂ représente un atome d'hydrogène ou un groupe OH,
R₃ représente un groupe : 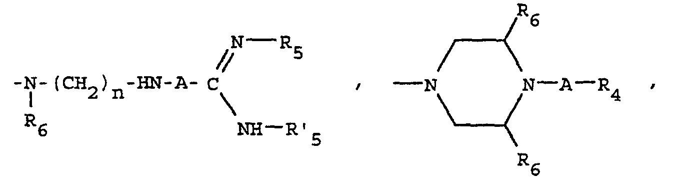 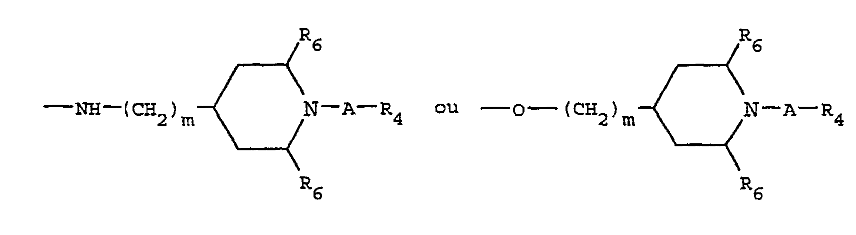 où
A représente une liaison simple ou un groupe -CO-(CH₂)ₚ-NH-
R₄ représente un atome d'hydrogène ou un groupe 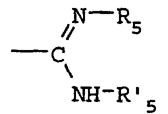
R₅ et R'₅, identiques ou différents, représentent chacun un atome d'hydrogène ou un groupe alkyle en C₁-C₆ linéaire, ramifié ou cyclisé,
R₆ représente un atome d'hydrogène ou un groupe alkyle en C₁-C₃ linéaire ou ramifié,
m est un nombre ayant pour valeur 0, 1 ou 2,
n est un nombre ayant pour valeur 2, 3 ou 4,
p est un nombre ayant pour valeur 1, 2 ou 3 ; et,
**(ii)** leurs sels d'addition.

2. Composé selon la revendication 1, **caractérisé en ce que**, dans la formule I, X est un atome de chlore.

3. Composé intermédiaire, utile pour la préparation des composés de formule I, **caractérisé en ce qu'**il répond à la formule : 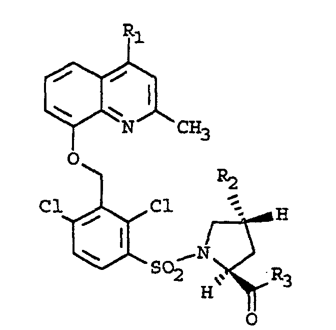 dans laquelle
R₁ représente CH₃,
R₂ représente H ou OH, et
R₃ représente OH ou OCH₃.

4. Procédé de préparation d'un composé de formule I, **caractérisé en ce qu'**il comprend les étapes consistant à :
1) faire réagir un composé de formule : 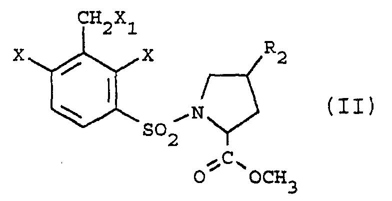
dans laquelle X et X₁ représentent chacun un halogène,
R₂ représente un atome d'hydrogène ou un groupe OH, et, le carbone porteur du groupe COOCH₃ et le carbone porteur du groupe
R₂ lorsque ce dernier n'est pas l'atome d'hydrogène, sont indépendamment l'un de l'autre de configuration (R,S), (R) ou (S),
avec un composé de formule : 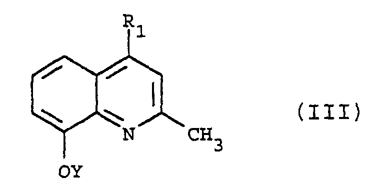 dans laquelle :
R₁ représente un atome d'hydrogène ou un groupe alkyle en C₁-C₃, et
Y représente un métal alcalin tel que le sodium ou le potassium,
dans un solvant anhydre, à température comprise entre 0 et 50° C, pendant 0,5 à 5 heures, pour obtenir un composé de formule : 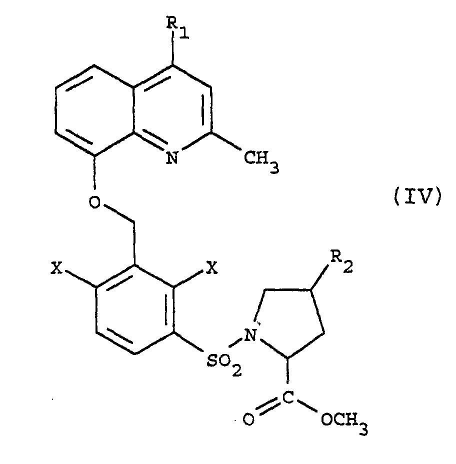 dans laquelle X, R₁ et R₂ conservent la même signification que dans les composés de départ, et les atomes de carbone porteurs de substituants COOCH₃ et R₂ conservent la même configuration que dans le composé II ci-dessus ;
2) soumettre le composé IV, ainsi obtenu, à une hydrolyse alcaline de la liaison ester, par action d'une solution aqueuse d'hydroxyde métallique (notamment NaOH) dans un solvant inerte, notamment le diméthoxyéthane, à une température comprise entre 10 et 50° C, pendant 1 à 30 heures pour obtenir, après acidification, le composé acide de formule : 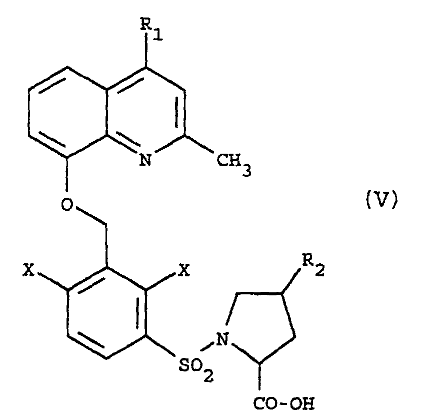 dans laquelle X, R₁ et R₂ conservent la même signification que dans le composé IV ci-dessus ;
3) faire réagir l'acide V, ainsi obtenu, avec un alcool ou une amine de formule: 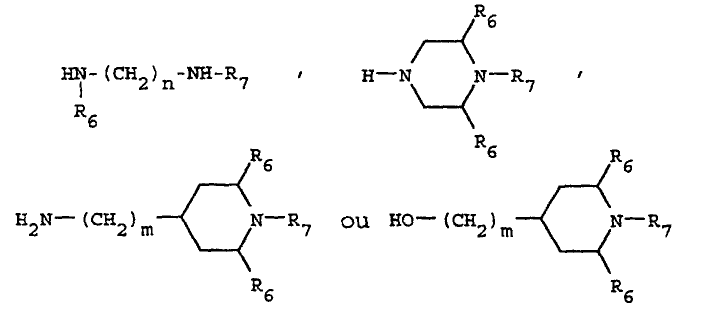 où
m représente 0, 1 ou 2,
n représente 2, 3 ou 4,
R₆ représente un atome d'hydrogène ou un groupe alkyle en C₁-C₃,
R₇ représente un groupe amino-protecteur notamment le groupe (1,1-diméthyléthoxy)carbonyle (Boc), ou, dans certains cas, un atome d'hydrogène,
dans un solvant, en présence d'un ou plusieurs activateurs, à une température proche de la température ambiante, pendant 2 à 50 heures, pour obtenir un composé de formule : 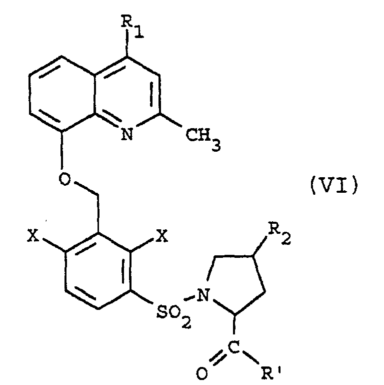 dans laquelle :
X, R₁ et R₂ conservent la même signification que ci-dessus et R' représente l'un des groupes: 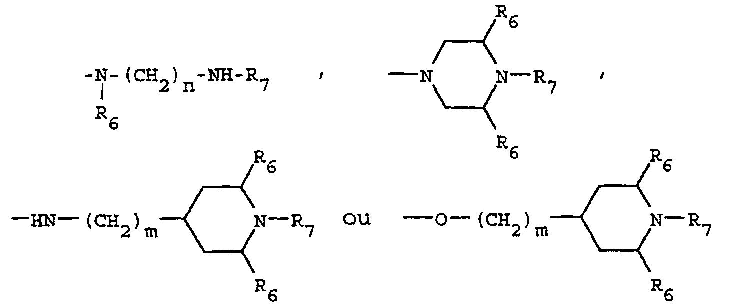 où m, n, R₆ et R₇ conservent la même signification que ci-dessus ;
4) si nécessaire, c'est-à-dire lorsque R₇ représente un groupe amino-protecteur, effectuer la déprotection du composé de formule VI, ainsi obtenu à l'étape précédente, par réaction dudit composé de formule VI avec un acide, éventuellement en présence d'un capteur de radicaux libres, et éventuellement dans un solvant, à une température proche de l'ambiante et pendant 1 à 20 heures, pour obtenir le composé de formule générale VI décrit ci-dessus où R₇ représente un atome d'hydrogène (correspondant au composé de formule I où A est une liaison simple et R₄ un atome d'hydrogène) ;
5) si nécessaire, faire réagir le composé de formule VI, ainsi obtenu, où R₇ est un atome d'hydrogène, avec un composé de formule :
R₈-NH-(CH₂)ₚ-COOH
dans laquelle p représente un nombre égal à 1, 2 ou 3, et
R₈ représente un groupe amino-protecteur,
dans des conditions de réaction analogues à celles décrites à l'étape 3 ci-dessus, Dour obtenir un composé de formule : 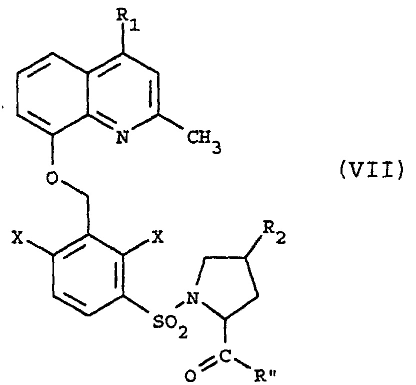 dans laquelle X, R₁ et R₂ conservent la même signification que ci-dessus et R" représente un groupe 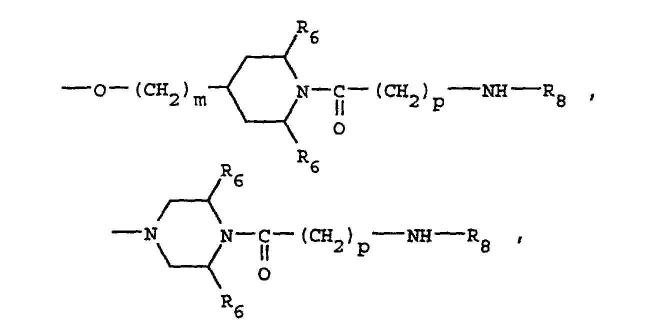 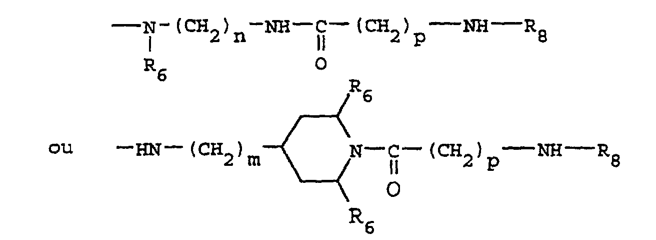 où m, n, p, R₆ et R₈ conservent la même que ci-dessus ;
6) faire réagir le composé de formule VII, obtenu selon l'étape 5 ci-dessus, dans des conditions analogues à celles décrites à l'étape 4 ci-dessus, de façon à remplacer le groupe amino-protecteur R₈ par un atome d'hydrogène et obtenir un composé de formule : 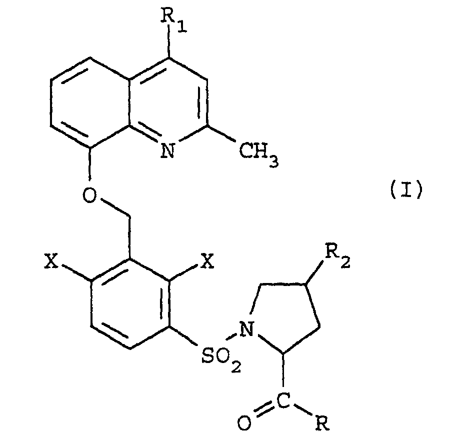 dans laquelle X, R₁ et R₂ conservent la même signification que ci-dessus et R représente un groupe 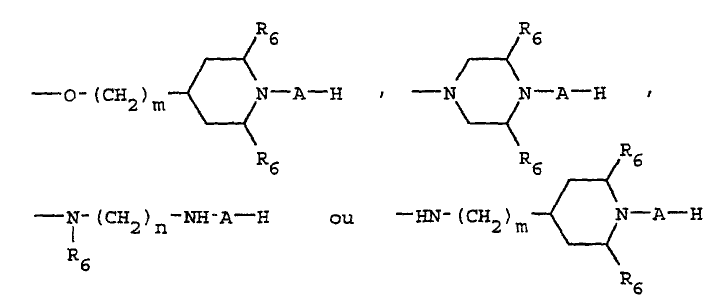 où m, n et R₆ conservent la même signification que ci-dessus et A représente le groupe -CO- (CH₂)ₚ-NH-, où p est un nombre égal à 1, 2 ou 3 ;
7) si nécessaire, faire réagir un composé de formule I obtenu à l'une des étapes 4 ou 6 ci-dessus, où A représente une liaison simple ou un groupe de formule : 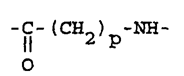 avec un composé de formule 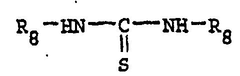 dans laquelle R₈ représente un groupe amino-protecteur de type oxycarbonyle,
dans un solvant, en présence d'une base et en présence de chlorure mercurique, à une température comprise entre 0 et 30 °C, pendant 1 à 6 heures, pour obtenir le composé de formule : 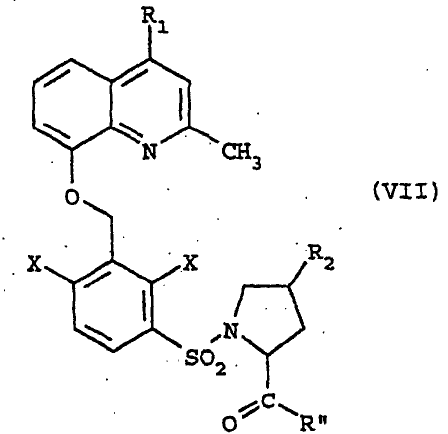 dans laquelle R₁, R₂ et X conservent la même signification que ci-dessus et R" représente un groupe 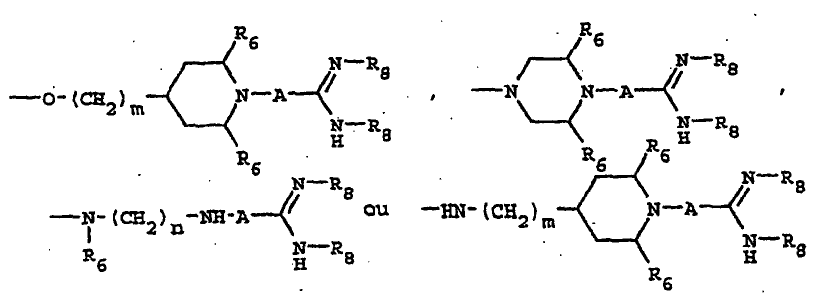
où A représente une liaison simple ou le groupe -CO-(CH₂)ₚ-NH-,
et n, m, p, R₆ et R₈ conservent la même signification que ci-dessus ; et,
8) déprotéger le composé de formule VII, ainsi obtenu selon l'étape 7, suivant une réaction analogue à celle de l'étape 6 ci-dessus, pour remplacer le groupe amino-protecteur R₈ par un atome d'hydrogène et obtenir ainsi un composé de formule I selon l'invention où R₄ représente un groupe -C(=NR₅)NHR'₅ et R₅ et R'₅ représentent chacun un atome d'hydrogène. formule I selon l'invention où R₄ représente un groupe -C(=NR₅)NHR'₅ et R₅ et R'₅ représentent chacun un atome d'hydrogène.

5. Procédé selon la revendication 4, **caractérisé en ce que** l'étape 7 est remplacée par l'étape qui consiste à :
7a) faire réagir le chlorhydrate d'un composé de formule I obtenu à l'une des étapes 4 ou 6 selon la revendication 4, avec un composé de formule :
R₅-N=C=N-R'₅
où R₅ et R'₅ représentent chacun un groupe alkyle en C₁-C₆ linéaire, ramifié ou cyclisé,
dans un solvant inerte, pour obtenir un composé de formule I, dans laquelle R₅ et R'₅ représentent chacun un groupe alkyle en C₁-C₆ linéaire, ramifié ou cyclisé.

6. Utilisation d'une substance choisie parmi l'ensemble constitué par les composés de formule I et leurs sels d'addition non-toxiques selon la revendication 1, pour l'obtention d'un médicament destiné à une utilisation en thérapeutique vis-à-vis d'états pathologiques impliquant la bradykinine ou ses homologues.

7. Utilisation selon la revendication 6, d'une substance choisie parmi l'ensemble constitué par les composés de formule I et leurs sels d'addition non-toxiques selon la revendication 1, pour l'obtention d'un médicament destiné à une utilisation en thérapeutique pour le traitement d'états douloureux ou inflammatoires.

8. Composition thérapeutique **caractérisée en ce qu'**elle renferme, en association avec un excipient physiologiquement acceptable, au moins un ingrédient actif choisi parmi l'ensemble constitué par les composés de formule I et leurs sels d'addition non-toxiques selon la revendication 1.

9. Utilisation d'un composé selon la revendication 1 en tant que réactif de dosage de la bradykinine ou du récepteur B₂ de la bradykinine.

10. Composé intermédiaire pour la synthèse des composés de formule I dans laquelle R₃ est le groupe 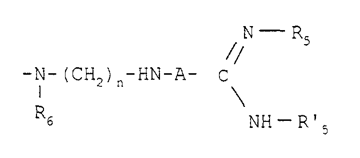 où A, R₅ et R'₅ sont définis comme dans la formule I de ladite revendication 1, n est 2, 3 ou 4 et R₆ est H ou alkyle en C₁-C₃,
ledit composé intermédiaire étant **caractérisé en ce qu'**il est choisi parmi l'ensemble constitué par les :
1-[[3-[(2-méthylquinolin-8-yl)oxyméthyl] -2,4-dichlorophényl]sulfonyl]-N-(2-aminoéthyl)-2-(S)-pyrrolidinecarboxamide,
1-[[3-[(2,4-diméthylquinolin-8-yl)oxyméthyl]-2,4-dichlorophényl]sulfonyl]-N-(2-aminoéthyl)-2-(S)-pyrrolidinecarboxamide,
1-[[3-[(2-méthylquinolin-8-yl)oxyméthyl]-2,4-dichlorophényl]sulfonyl]-N-méthyl-N-(2-aminoéthyl)-2-(S)-pyrrolidinecarboxamide,
1-[[3-[(2-méthylquinolin-8-yl)oxyméthyl]-2,4-dichlorophényl]sulfonyl]-N-(4-aminobutyl)-2-(S)-pyrrolidinecarboxamide,
1-[[3-[(2,4-diméthylquinolin-8-yl)oxyméthyl]-2,4-dichlorophényl]sulfonyl]-N-(3-aminopropyl)-2-(S)-pyrrolidinecarboxamide, et
1-[[3-[(2-méthylquinolin-8-yl)oxyméthyl]-2,4-dichlorophényl]sulfonyl]-N-(4-aminobutyl)-2-(S)-pyrrolidinecarboxamide.

## Patentansprüche

1. N-Benzolsulfonylpyrrolidin-Verbindung, **dadurch gekennzeichnet, dass** sie aus der Gesamtheit ausgewählt ist, die von
a) Verbindungen mit der Formel 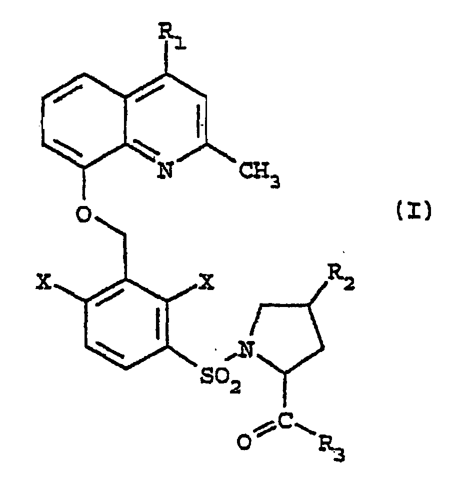 in welcher
X ein Halogenatom,
R₁ ein Wasserstoffatom oder eine C₁- bis C₃-Alkylgruppe mit geradkettiger oder verzweigter Kohlenwasserstoffkette,
R₂ ein Wassers
R₃ eine Gruppe 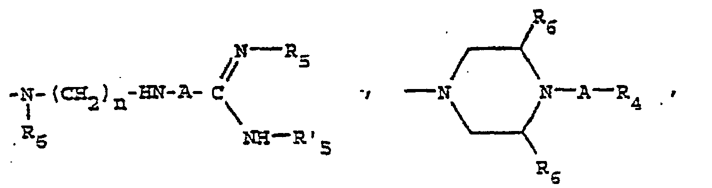 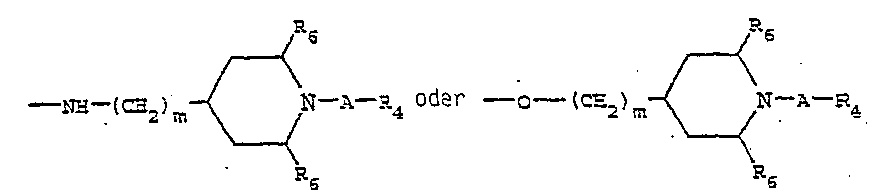 bedeutet, worin
A eine Einfachbindung oder eine -CO-(CH₂)ₚ-NH-Gruppe und
R₄ ein Wasserstoffatom oder eine Gruppe 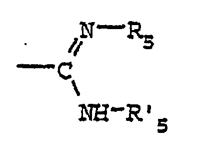 bedeutet und
R₅ und R₅', die gegebenenfalls verschieden sind, jeweils ein Wasserstoffatom oder eine geradkettige, verzweigte bzw. cyclische C₁- bis C₆-Alkylgruppe bedeuten und
R₆ ein Wasserstoffatom oder eine geradkettige bzw. verzweigte C₁- bis C₃-Alkylgruppe bedeutet, wobei
m ganzzahlig mit 0, 1 oder 2,
n ganzzahlig mit 2, 3 oder 4 und
p ganzzahlig mit 1, 2 oder 3 ist, und
b) ihren Additionssalzen gebildet wird.

2. Verbindung nach Anspruch 1, **dadurch gekennzeichnet, dass** in der Formel I X ein Chloratom bedeutet.

3. Zwischenverbindung, die für die Herstellung der Verbindungen mit der Formel I nützlich ist, **dadurch gekennzeichnet,** 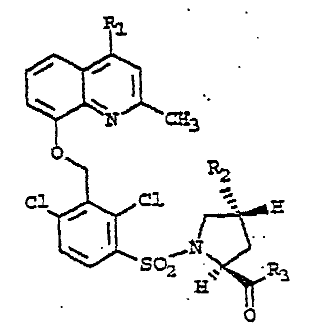 entspricht, in welcher
R₁ CH₃,
R₂ H oder OH und
R₃ OH oder OCH₃ bedeutet.

4. Verfahren zur Herstellung einer Verbindung mit der Formel I, **dadurch gekennzeichnet, dass** es die Stufen umfasst, die aus
1) 0,5 bis 5 Stunden langes Umsetzen einer Verbindung mit der Formel 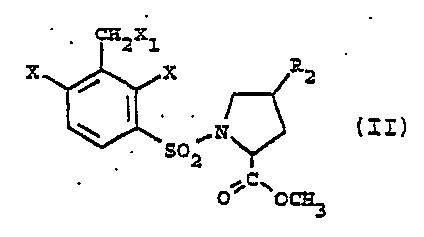 in welcher X und X₁ jeweils ein Halogen bedeuten und
R₂ ein Wasserstoffatom oder eine OH-Gruppe bedeutet, wobei das Kohlenstoffträgeratom der COOCH₃-Gruppe und das Kohlenstoffträgeratom der R₂-Gruppe, wenn letztere kein Wasserstoffatom ist, unabhängig voneinander die Konfiguration (R,S), (R) oder (S) haben,
mit einer Verbindung mit der Formel 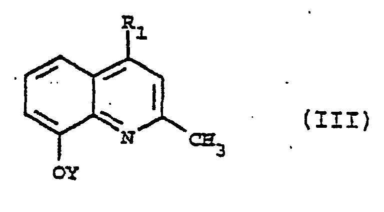 in welcher
R₁ ein Wasserstoffatom oder eine C1- bis C₃-Alkylgruppe und
Y ein Alkalimetall wie Natrium oder Kalium bedeutet,
in einem wasserfreien Lösungsmittel bei einer Temperatur von 0 bis 50 °C, um eine Verbindung mit der Formel 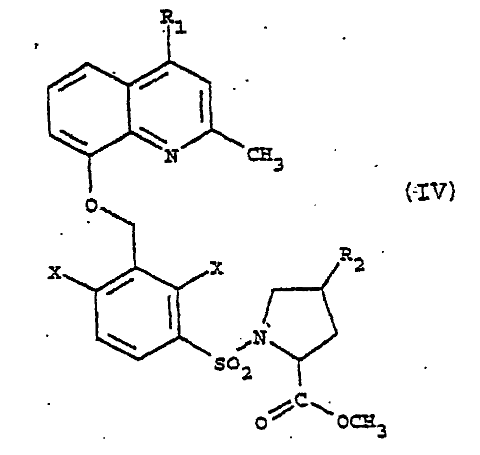 zu erhalten, in welcher X, R₁ und R₂ dieselbe Bedeutung wie in den Ausgangsverbindungen und die Kohlenstoffträgeratome der Substituenten COOCH₃ und R₂ dieselbe Konfiguration wie in obiger Verbindung II beibehalten,
2) 1 bis 30 Stunden langes Unterwerfen der so erhaltenen Verbindung IV einer alkalischen Hydrolyse der Esterbindung durch Einwirkung einer wässrigen Metallhydroxidlösung (insbesondere NaOH) in einem inerten Lösungsmittel, insbesondere Dimethoxyethan, bei einer Temperatur von 10 bis 50 °C, um nach Ansäuern die saure Verbindung mit der Formel 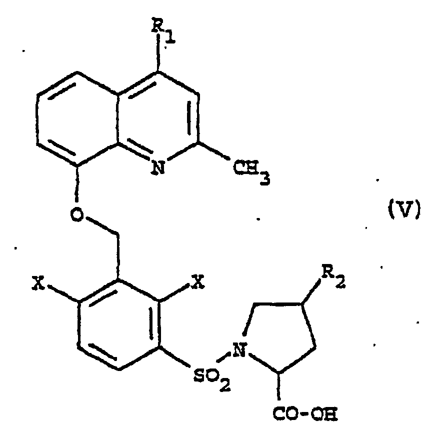 zu erhalten, in wclcher X, R₁ und R₂ dieselbe Bedeutung wie in obiger Verbindung IV beibehalten,
3) 2 bis 50 Stunden langes Umsetzen der so erhaltenen Säure V mit einem Alkohol oder Amin mit der Formel 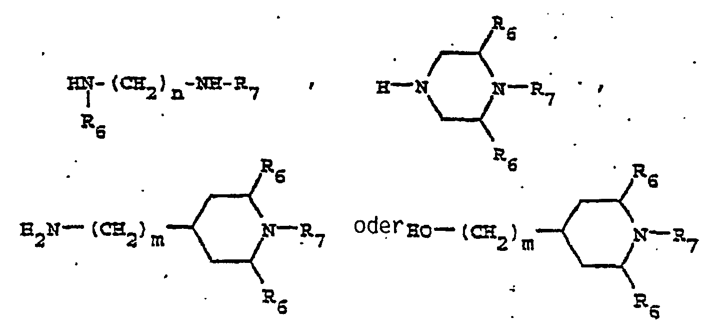 worin
m 0, 1 oder 2,
n 2, 3 oder 4,
R₆ ein Wasserstoffatom oder eine C₁- bis C₃-Alkylgruppe und
R₇ eine Aminoschutzgruppe, insbesondere die (1,1-Dimethylethoxy)carbonyl-Gruppe (Boc) oder in bestimmten Fällen ein Wasserstoffatom bedeutet,
in einem Lösungsmittel in Gegenwart eines oder mehrerer Aktivatoren bei einer Temperatur in der Nähe der Umgebungstemperatur, um eine Verbindung mit der Formel 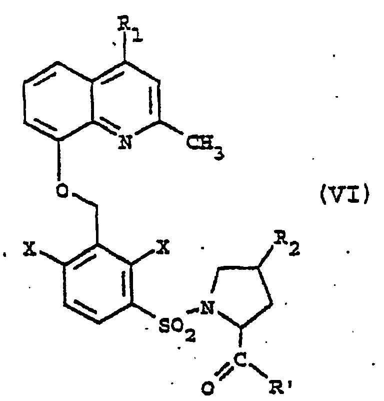 zu erhalten, in welcher
X, R₁ und R₂ dieselbe Bedeutung wie weiter oben beibehalten und R' eine der Gruppen 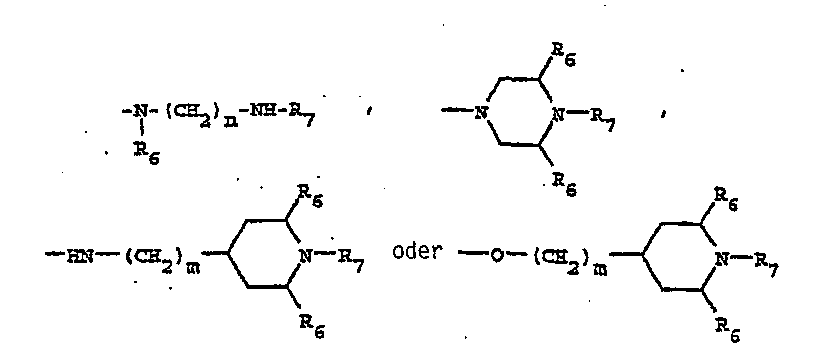 bedeutet, worin m, n, R₆ und R₇ dieselbe Bedeutung wie weiter oben beibehalten,
4) erforderlichenfalls, d.h., wenn R₇ eine Aminoschutzgruppe bedeutet, Entfernen der Schutzgruppe aus der so in der vorhergehenden Stufe erhaltenen Verbindung mit der Formel VI durch 1 bis 20 Stunden lange Umsetzung der Verbindung mit der Formel VI mit einer Säure, gegebenenfalls in Gegenwart eines Fängers für freie Radikale und gegebenenfalls in einem Lösungsmittel bei einer Temperatur in der Nähe der Umgebungstemperatur, um die zuvor beschriebene Verbindung mit der allgemeinen Formel VI zu erhalten, worin R₇ ein Wasserstoffatom (entspricht der Verbindung mit der Formel I, worin A eine Einfachbindung und R₄ ein Wasserstoffatom bedeutet) bedeutet,
5) erforderlichenfalls Umsetzen der so erhaltenen Verbindung mit der Formel VI, worin R₇ ein Wasserstoffatom bedeutet, mit einer Verbindung mit der Formel
R₈-NH-(CH₂)ₚ-COOH,
in welcher p eine Zahl von gleich 1, 2 oder 3 und R₈ eine Aminoschutzgruppe bedeutet, unter Reaktionsbedingungen, die den in obiger Stufe 3 beschriebenen analog sind, um eine Verbindung mit der Formel 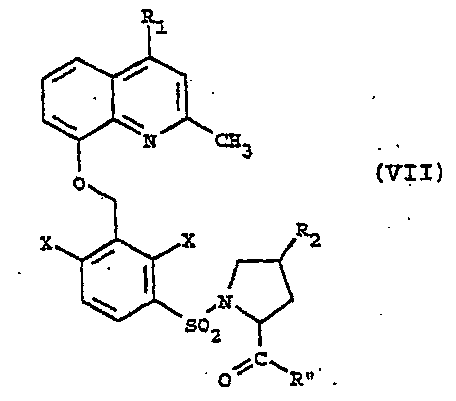 zu erhalten, in welcher X, R₁ und R₂ dieselbe Bedeutung wie weiter oben beibehalten und R" eine Gruppe 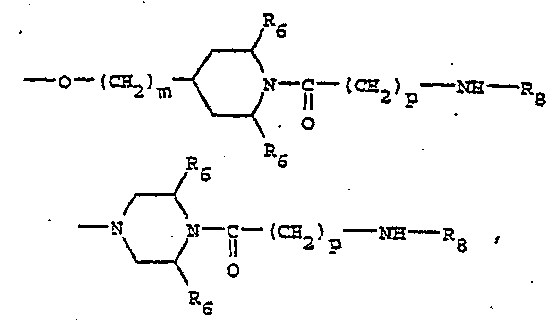 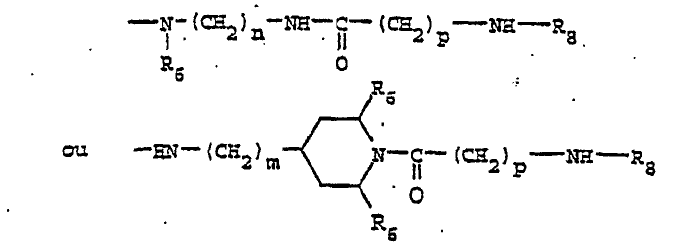 bedeutet, worin m, n, p, R₆ und R₈ dieselbe Bedeutung wie zuvor beibehalten,
6) Umsetzen der in der Stufe 5 erhaltenen Verbindung mit der Formel VII unter Bedingungen, die den in obiger Stufe 4 beschriebenen analog sind, derart, **dass** die Aminoschutzgruppe R₈ durch ein Wasserstoffatom ersetzt und eine Verbindung mit der Formel 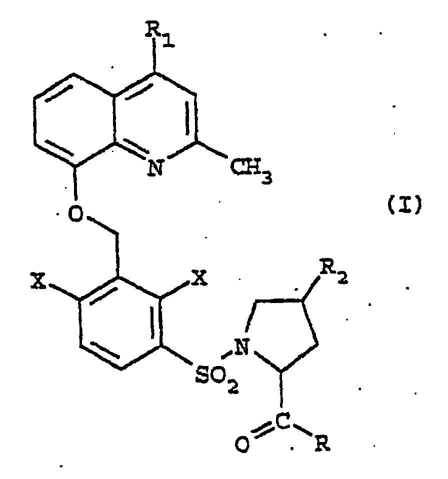 erhalten wird, in welcher X, R₁ und R₂ dieselbe Bedeutung wie zuvor beibehalten und R eine Gruppe 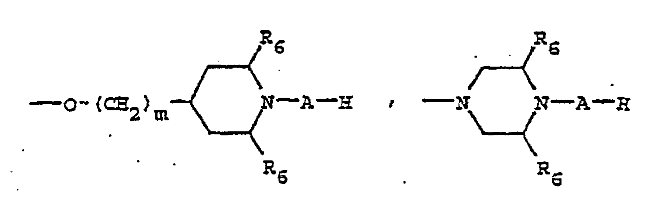 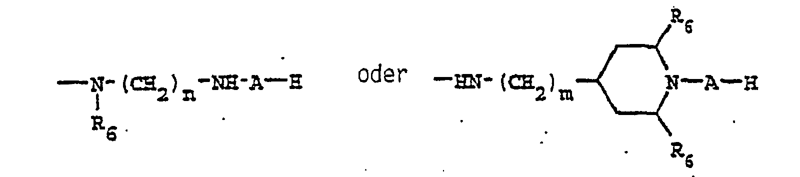 bedeutet, worin m, n und R₆ dieselbe Bedeutung wie zuvor beibehalten und A die -CO-(CH₂)ₚ-NH-Gruppe bedeutet, wobei p eine ganze Zahl von gleich 1, 2 oder 3 bedeutet,
7) erforderlichenfalls 1 bis 6 Stunden langes Umsetzen einer Verbindung mit der Formel I, die in einer der obigen Stufen 4 oder 6 erhalten wurde, worin A eine Einfachbindung oder eine Gruppe mit der Formel 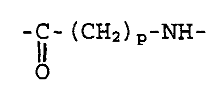 bedeutet, mit einer Verbindung mit der Formel 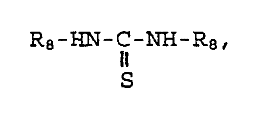 in welcher R₈ eine Aminoschutzgruppe vom Typ Oxycarbonyl bedeutet, in einem Lösungsmittel in Gegenwart einer Base und von Quecksilberchlorid bei einer Temperatur von 0 bis 30 °C, um die Verbindung mit der Formel 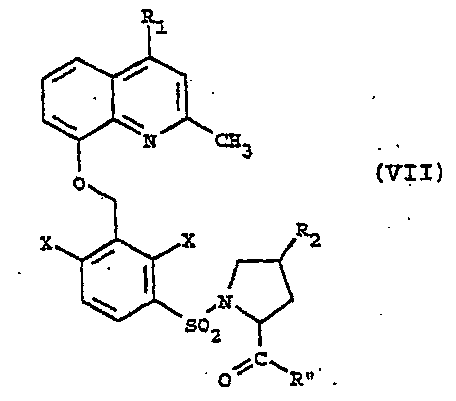 zu erhalten, in welcher R₁, R₂ und X dieselbe Bedeutung wie weiter oben beibehalten und R" eine Gruppe 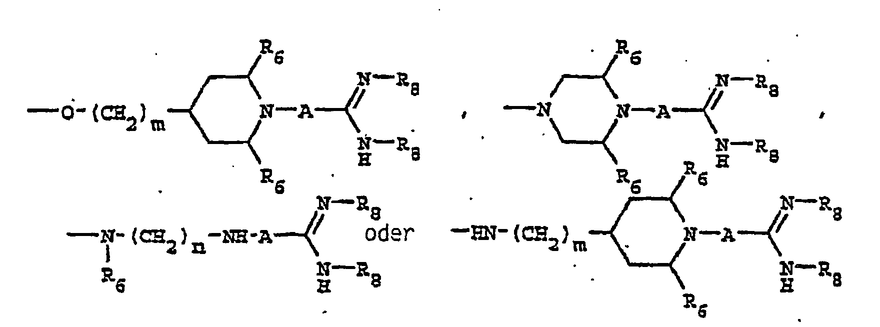 bedeutet, worin A eine Einfachbindung oder die -CO-(CH₂)ₚ-NH-Gruppe bedeutet und n, m, p, R₆ und R₈ dieselbe Bedeutung wie weiter oben beibehalten, und
8) Entfernen der Schutzgruppe aus der in Stufe 7 erhaltenen Verbindung VII durch eine Reaktion, die analog der in der obigen Stufe 6 ist, um die Aminoschutzgruppe R₈ durch ein Wasserstoffatom zu ersetzen und so eine Verbindung zu erhalten, bestehen.

5. Verfahren nach Anspruch 4, **dadurch gekennzeichnet, dass** die Stufe 7 durch eine Stufe ersetzt wird, die darin besteht,
7a) das Chlorhydrat einer Verbindung mit der Formel I, die in einer der Stufen 4 oder 6 gemäß Anspruch 4 erhalten wurde, mit einer Verbindung mit der Formel
R₅-N=C=N-R₅' ,
worin R₅ und R₅' jeweils eine geradkettige, verzweigte oder cyclische C₁- bis C₆-Alkylgruppe bedeutet, in einem inerten Lösungsmittel umzusetzen, um eine Verbindung mit der Formel I zu erhalten, in welcher R₅ und R₅' jeweils eine geradkettige, verzweigte oder cyclische C1- bis C6-Alkylgruppe bedeutet.

6. Verwendung einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei Erkrankungen vorgesehen ist, an denen Bradykinin oder dessen Homologen beteiligt sind.

7. Verwendung nach Anspruch 6 einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei der Behandlung von Schmerz- oder Entzündungszuständen vorgesehen ist.

8. Therapeutische Zusammensetzung, **dadurch gekennzeichnet, dass** sie zusammen mit einem physiologisch verträglichen Arzneimittelträger mindestens einen Wirkstoff enthält, der aus der Gesamtheit ausgewählt ist, die von den Verbindungen mit der Formel I und deren nichttoxischen Additionssalzen nach Anspruch 1 gebildet wird.

9. Verwendung einer Verbindung nach Anspruch 1 als Reagenz für die quantitative Bestimmung des Bradykinins oder des B₂-Rezeptors für das Bradykinin.

10. Zwischenverbindung für die Synthese der Verbindungen mit der Formel I, in welcher R₃ die Gruppe 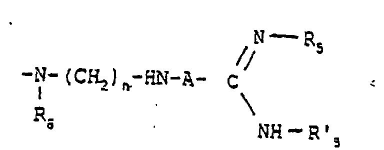 bedeutet, worin A, R₅ und R₅' wie in der Formel I des Anspruchs 1 definiert sind und n 2, 3 oder 4 und R₆ H oder eine C₁- bis C₃-Alkylgruppe bedeutet, wobei die Zwischenverbindung **dadurch gekennzeichnet ist, dass** sie von der Gesamtheit ausgewählt ist, die aus
1- [[3- [(2-Methylchinolin-8-yl)oxymethyl]-2,4-dichlorphenyl]sulfonyl]-N-(2-aminoethyl)-2-(S)-pyrrolidincarboxamid,
1-[[3-[(2,4-Dimethylchinolin-8-yl)oxymethyl]-2,4-dichlorphenyl]sulfonyl]-N-(2-aminoethyl)-2-(S)-pyrrolidincarboxamid,
1-[[3-[(2-Methylchinolin-8-yl)oxymethyl]-2,4-dichlorphenyl]sulfonyl]-N-methyl-N-(2-aminoethyl) -2-(S)-pyrrolidincarboxamid,
1-[[3-[(2-Methylchinolin-8-yl)oxymethyl]-2,4-dichlorphenyl]sulfonyl]-N-(4-aminobutyl)-2-(S)-pyrrolidincarboxamid,
1-[[3-[(2,4-Dimethylchinolin-8-yl)oxymethyl]-2,4-dichlorphenyl]sulfonyl]-N-(3-aminopropyl)-2-(S)-pyrrolidincarboxamid und
1-[[3-[(2-Methylchinolin-8-yl)oxymethyl]-2,4-dichlorphenyl]sulfonyl]-N-(4-aminobutyl)-2-(S)-pyrrolidincarboxamid
besteht.

## Claims

1. N-Benzenesulfonylpyrrolidine compound, **characterized in that** it is selected from the group consisting of:
(i) the compounds of the formula 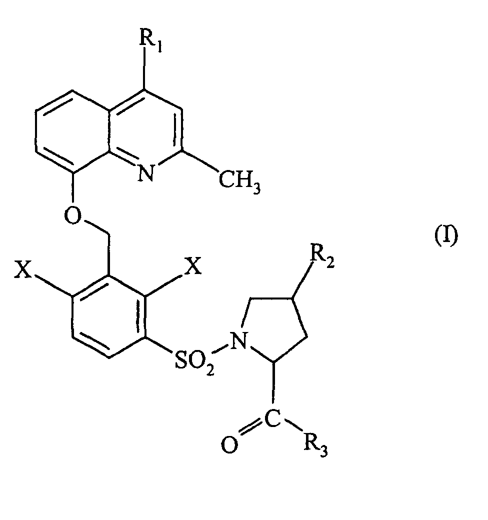 in which:
X is a halogen atom,
R₁ is a hydrogen atom or a C₁-C₃ alkyl group with a linear or branched hydrocarbon chain,
R₂ is a hydrogen atom or an OH group, and
R₃ is a group 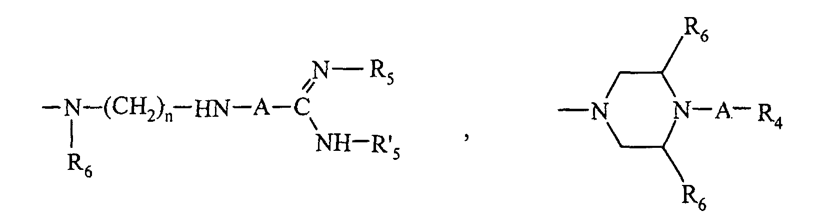 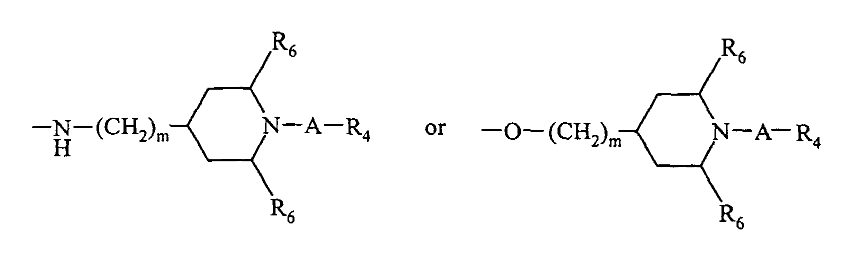
in which:
A is a single bond or a group -CO-(CH₂)ₚ-NH-,
R₄ is a hydrogen atom or a group 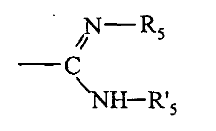
R₅ and R'₅ which are identical or different, are each a hydrogen atom or a linear, branched or cyclized C₁-C₆ alkyl group,
R₆ is a hydrogen atom or a linear or branched C₁-C₃ alkyl group,
m is a number with a value of 0, 1, or 2,
n is a number with a value of 2, 3 or 4,
p is a number with a value of 1, 2 or 3; and
(ii) their addition salts.

2. Compound according to claim 1, **characterized in that** X in formula I is a chlorine atom.

3. Intermediate useful for the preparation of the compounds of formula I, **characterized in that** it has the formula 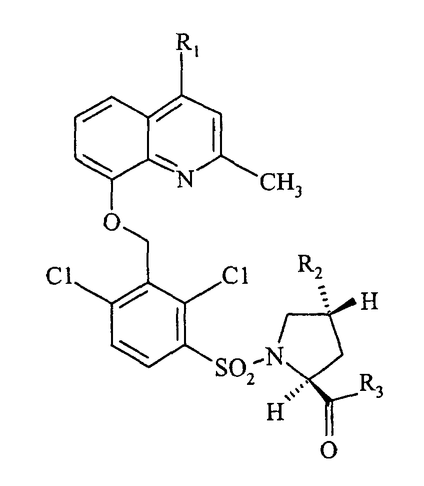 in which:
R₁ is CH₃,
R₂ is H or OH, and
R₃ is OH or OCH₃.

4. Process for the preparation of a compound of formula I, **characterized in that** it comprises the steps which consist in:
1) reacting a compound of the formula 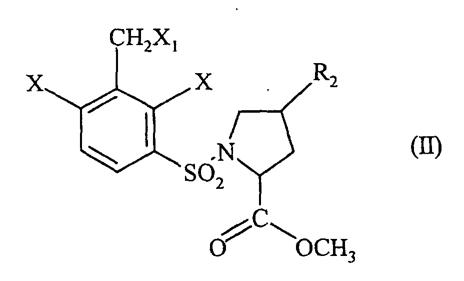
in which X and X₁ are each a halogen,
R₂ is a hydrogen atom or an OH group, and the carbon carrying the group COOCH₃ and the carbon carrying the group R₂, when the latter is not the hydrogen atom, independently of one another have the (R,S), (R) or (S) configuration, with a compound of the formula 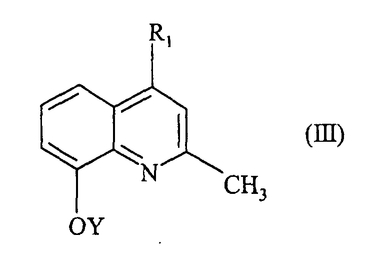
in which:
R₁ is a hydrogen atom or a C₁-C₃ alkyl group, and
Y is an alkali metal such as sodium or potassium, in an anhydrous solvent, at a temperature of between 0 and 50°C, for 0.5 to 5 hours, to give a compound of the formula 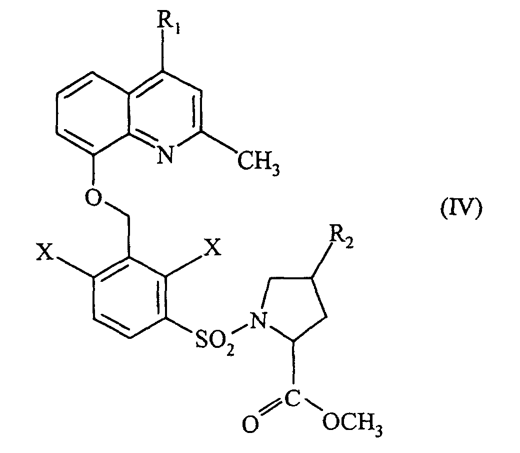
in which X, R₁ and R₂ are as defined in the starting compounds and the carbon atoms carrying the substituents COOCH3 and R₂ retain the same configuration as in the compound II above;
2) subjecting the resulting compound IV to alkaline hydrolysis of the ester linkage by reaction with an aqueous solution of a metal hydroxide (especially NaOH) in an inert solvent, especially dimethoxyethane, at a temperature of between 10 and 50°C, for 1 to 30 hours, to give, after acidification, the acid compound of the formula 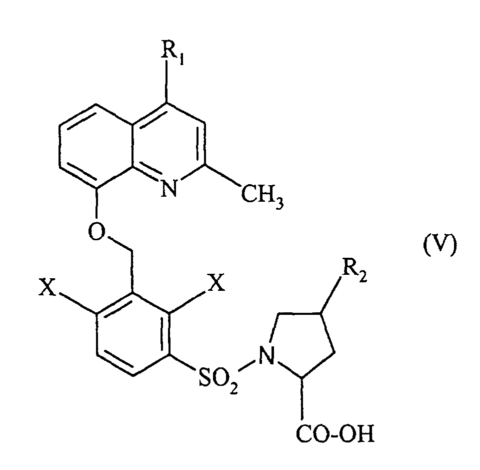 in which X, R₁ and R₂ are as defined in the compound IV above;
3) reacting the resulting acid V with an alcohol or an amine of the formula 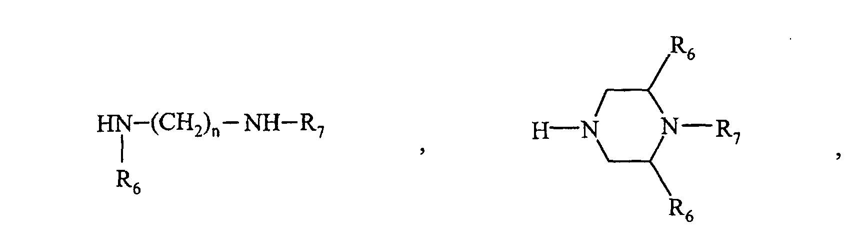 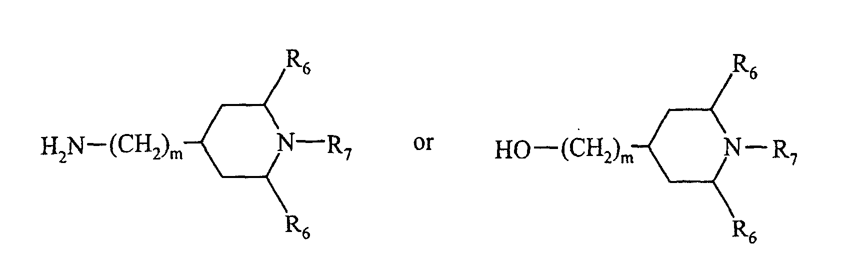 in which:
m is 0, 1 or 2,
n is 2, 3 or 4,
R₆ is a hydrogen atom or a C₁-C₃ alkyl group, and
R₇ is an amino-protecting group, especially the (1,1-dimethylethoxy)carbonyl (Boc) group, or, in certain cases, a hydrogen atom,
in a solvent, in the presence of one or more activators, at a temperature close to room temperature, for 2 to 50 hours, to give a compound of the formula 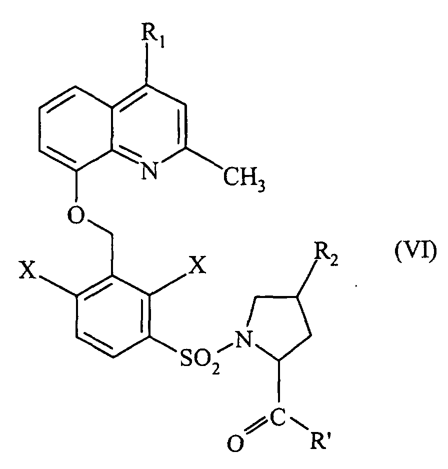 in which:
X, R₁ and R₂ are as defined above and R' is one of the following groups: 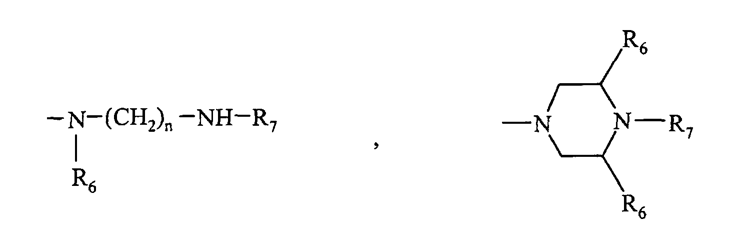 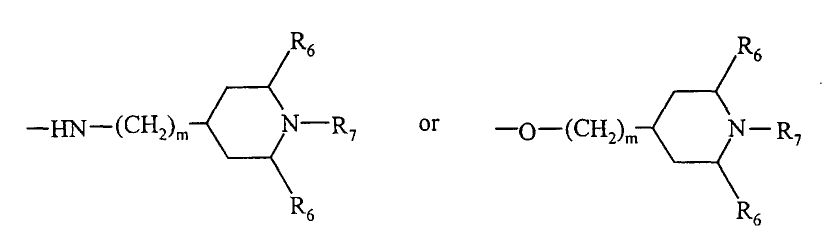 in which m, n, R₆ and R₇ are as defined above;
4) if necessary, i.e. when R₇ is an amino-protecting group, deprotecting the compound of formula VI thus contained in the previous step by reacting said compound of formula VI with an acid, optionally in the presence of a free radical scavenger, and optionally in a solvent, at a temperature close to room temperature, for 1 to 20 hours, to give the compound of general formula VI described above in which R₇ is a hydrogen atom (corresponding to the compound of formula I in which A is a single bond and R₄ is a hydrogen atom);
5) if necessary, reacting the resulting compound of formula VI in which R₇ is a hydrogen atom with a compound of the formula
R₈-NH-(CH₂)ₚ-COOH
in which p is a number equal to 1, 2 or 3, and
R₈ is an amino-protecting group, under reaction conditions analogous to those described in step 3 above, to give a compound of the formula 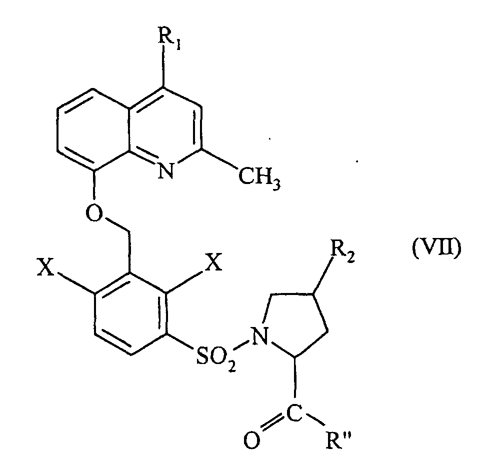 in which X, R₁ and R₂ are as defined above and R" is a group 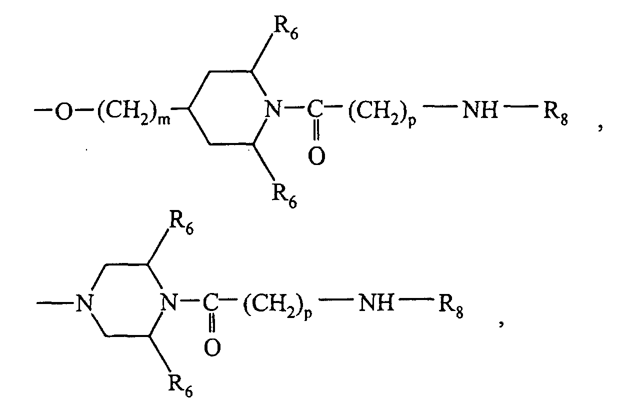 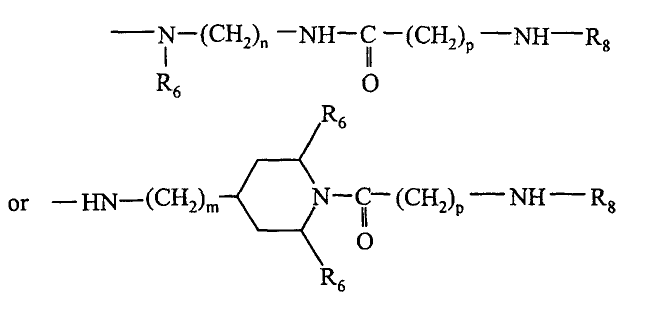 in which m, n, p, R₆ and R₈ are as defined above;
6) reacting the compound of formula VII obtained according to step 5 above, under conditions analogous to those described in step 4 above, so as to replace the amino-protecting group R₈ with a hydrogen atom to give a compound of the formula 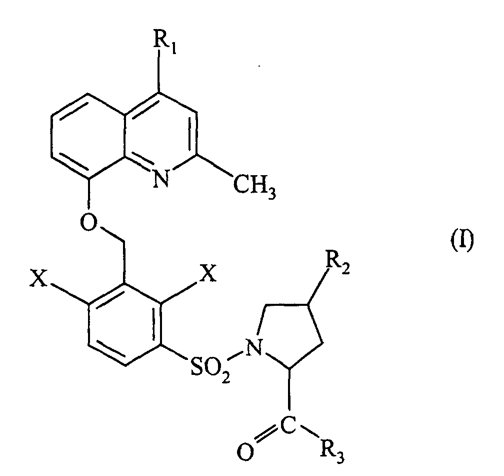 in which X, R₁ and R₂ are as defined above and R is a group 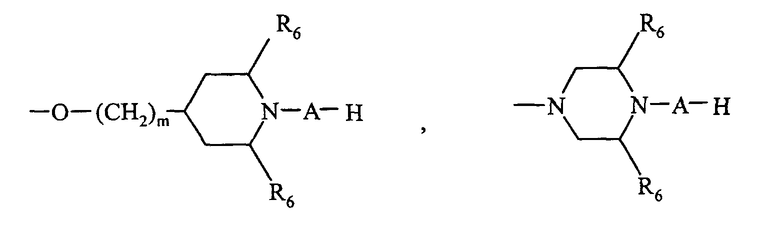 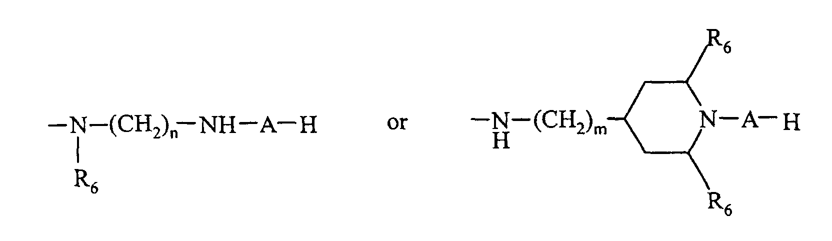 in which m, n, and R₆ are as defined above and A is the group -CO-(CH₂)ₚ-NH-, in which p is a number equal to 1, 2 or 3;
7) if necessary, reacting a compound of formula I obtained in one of steps 4 or 6 above, in which A is a single bond or a group of the formula 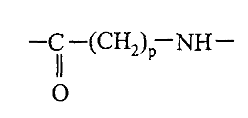 with a compound of the formula 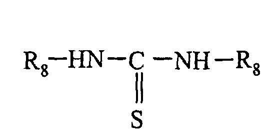 in which R₈ is an amino-protecting group of the oxycarbonyl type, in a solvent, in the presence of a base and in the presence of mercuric chloride, at a temperature of between 0 and 30°C, for 1 to 6 hours, to give the compound of the formula 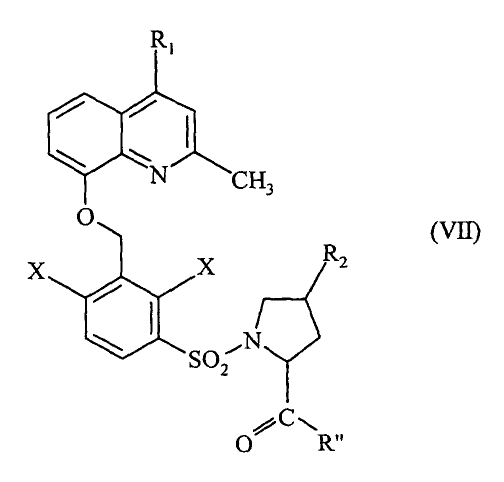 in which R₁, R₂ and X are as defined above and R" is a group 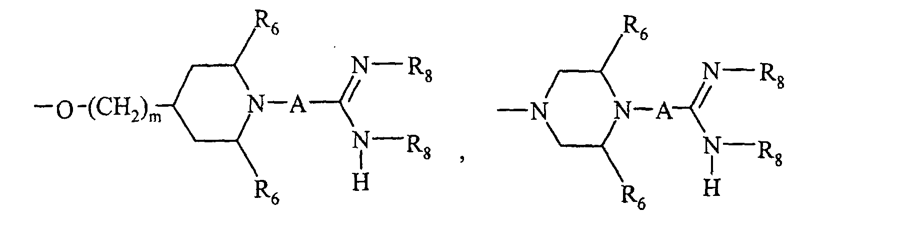 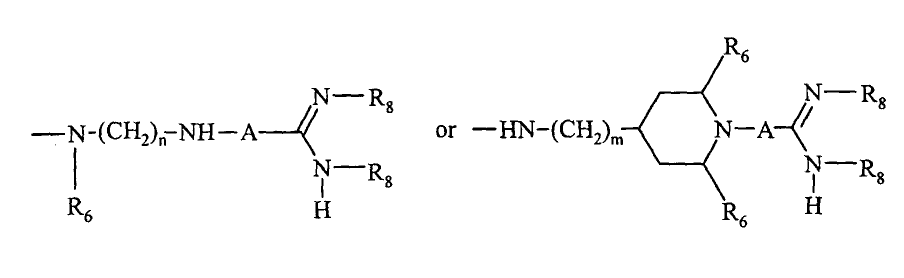 in which:
A is a single bond or the group -CO-(CH₂)ₚ-NH-, and
n, m, p, R₆ and R₈ are as defined above; and
8) deprotecting the compound of formula VII thus obtained according to step 7, by means of a reaction analogous to that of step 6 above, so as to replace the amino-protecting group R₈ with a hydrogen atom to give a compound of formula I according to the invention in which R₄ is a group -C(=NR₅)NHR'₅ and R₅ and R'₅ are each a hydrogen atom.

5. Process according to claim 4, **characterized in that** step 7 is replaced with the step which consists in:
7a) reacting the hydrochloride of a compound of formula I obtained in one of steps 4 or 6 according to claim 4 with a compound of the formula
R₅-N=C=N-R'₅
in which R₅ and R'₅ are each a linear, branched or cyclized C₁-C₆ alkyl group, in an inert solvent, to give a compound of formula I in which R₅ and R'₅ are each a linear, branched or cyclized C₁-C₆ alkyl group.

6. Use of a substance selected from the group consisting of the compounds of formula I and their non-toxic addition salts according to claim 1 for obtaining a drug intended for use in therapeutics to combat pathological conditions involving bradykinin or its homologs.

7. Use according to claim 6 of a substance selected from the group consisting of the compounds of formula I and their non-toxic addition salts according to claim 1 for obtaining a drug intended for use in therapeutics for the treatment of painful or inflammatory states.

8. Therapeutic composition, **characterized in that** it contains, in association with a physiologically acceptable excipient, at last one active ingredient selected from the group consisting of the compounds of formula I and their non-toxic addition salts according to claim 1.

9. Use of a compound according to claim 1 as reagent for determining bradykinin of the bradykinin B₂ receptor.

10. Intermediate useful for the preparation of the compounds of formula I, in which R₃ is the group 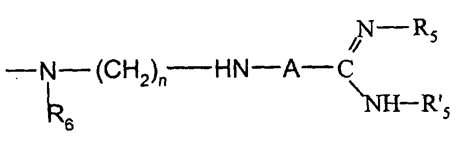 in which A, R₅ and R'₅ are as defined in formula I in claim 1, n is 2, 3 or 4 and R₆ is H or a C₁-C₃ alkyl group, said intermediate being **characterized in that** it is selected from the group consisting of:
1-[[3-[2-Methylquinolin-8-yl)oxymethyl]-2,4-dichlorophenyl]sulfonyl]-N-(2-aminoethyl)-2-(S)-pyrrolidinecarboxamide,
1-[[3-[2,4-Dimethylquinolin-8-yl)oxymethyl]-2,4-dichlorophenyl]sulfonyl]-N-(2-aminoethyl)-2-(S)-pyrrolidinecarboxamide,
1-[[3-[2-Methylquinolin-8-yl)oxymethyl]-2,4-dichlorophenyl]sulfonyl]-N-methyl-N-(2-aminoethyl)-2-(S)-pyrrolidinecarboxamide bistrifluoroacetate,
1-[[3-[2-Methylquinolin-8-yl)oxymethyl]-2,4-dichlorophenyl]sulfonyl]-N-(3-aminopropyl)-2-(S)-pyrrolidinecarboxamide dihydrochloride,
1-[[3-[2,4-Dimethylquinolin-8-yl)oxymethyl]-2,4-dichlorophenyl]sulfonyl]-N-(3-aminopropyl)-2-(S)-pyrrolidinecarboxamide dihydrochloride,
1-[[3-[2-Methylquinolin-8-yl)oxymethyl]-2,4-dichlorophenyl]sulfonyl]-N-(4-aminobutyl)-2-(S)-pyrrolidinecarboxamide dihydrochloride.
